# EUROPEAN PATENT APPLICATION

(11) **EP 4 397 322 A1**
(43) Date of publication of application: **10.07.2024**
(21) Application number: 22863569.4
(22) Date of filing: 01.09.2022
(51) Int. Cl.: A61K 51/04, A61K 31/547, C07D 265/14, A61P 35/00

(54) **INHIBITOR OF PROSTATE SPECIFIC MEMBRANE ANTIGEN AND PHARMACEUTICAL USE THEREOF**

(30) Priority: 01.09.2021 CN 202111018447; 30.12.2021 CN 202111649840; 06.05.2022 CN 202210488768
(71) Applicant: Tianjin Hengrui Medicine Co., Ltd., Tianjin 300303 (CN); Jiangsu Hengrui Pharmaceuticals Co., Ltd., Lianyungang, Jiangsu 222047 (CN)
(72) Inventor: WANG, Mengzhe, Tianjin 300303 (CN); ZHOU, Shunguang, Tianjin 300303 (CN); YU, Liang, Tianjin 300303 (CN); WANG, Lidong, Tianjin 300303 (CN); ZHAO, Libo, Tianjin 300303 (CN); SUN, Jiyun, Tianjin 300303 (CN); GUO, Feihu, Tianjin 300303 (CN); LI, Xin, Lianyungang, Jiangsu 222047 (CN)
(74) Representative: Regimbeau
(86) International application number: PCT/CN2022/116453
(87) International publication number: WO 2023/030434

(57) **Abstract**

An inhibitor of a prostate specific membrane antigen and a pharmaceutical use thereof. Specifically, the present solution belongs to the field of radiopharmaceuticals and relates to a compound represented by formula (IV) or a pharmaceutically acceptable salt thereof.

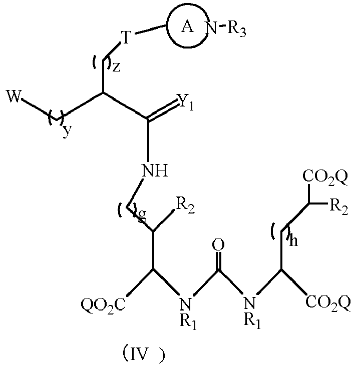

## Description

### TECHNICAL FIELD

The present disclosure pertains to the field of radiopharmaceuticals and particularly relates to an inhibitor of prostate specific membrane antigen (PSMA) and pharmaceutical use thereof.

### BACKGROUND

Prostate cancer (PCa) has now become the second most common cancer in males and is second only to lung cancer in terms of incidence and mortality, with nearly 1.6 million new cases worldwide each year. Although 366 thousand deaths were associated with prostate cancer in 2018, prostate cancer mortality has been decreasing in many developed countries, primarily due to the widespread use of prostate specific antigen (PSA) blood tests. PSA is thought to have revolutionized PCa screening as it is a valid indicator of relapses following an initial treatment such as radical prostatectomy (RP) or local radiotherapy (RT). This disease state, defined as biochemical relapse (BCR), is characterized by an increase in the PSA level following the initial treatment of PCa.

Over the last few decades, new diagnostic/prognostic tools, particularly imaging examinations, have been introduced in clinical practice to better support the diagnosis and treatment of prostate cancer in patients and to overcome some of the limitations of measuring PSA levels. Currently, clinical imaging methods include transrectal ultrasound (TRUS) for guiding biopsy and placing particles for brachytherapy, magnetic resonance imaging (MRI) and computed tomography (CT) for staging prostate cancer and detecting metastases and spread, and bone imaging for assessing bone metastases. These traditional imaging techniques are less sensitive and specific in detecting early/small recurrences or metastases, such as lymph node and sclerotic bone metastases. Over the last few years, radiological imaging methods and the use of radiopharmaceuticals have played a prominent role in the diagnosis and treatment of urogenital diseases, particularly PCa.

New detection means helpful in the staging, diagnosis, and classification of the disease are undoubtedly crucial for monitoring relapses and assessing efficacy. With the continued use of scientific discoveries and technological improvements, researchers have studied new biochemical pathways and cellular structures that may serve as targets for the treatment of the disease. Among them, prostate specific membrane antigen (PMSA) is increasingly important as a target for the specific action of drugs, especially radiopharmaceuticals.

PSMA, also known as folate hydrolase I (FOLH1) or glutamate carboxypeptidase II (GCPII), is a type II transmembrane glycoprotein of 750 amino acids, which is expressed to some extent in healthy human tissues, such as the lacrimal and salivary glands, the epididymis, the ovaries, the normal human prostatic epithelium, the central nervous system (CNS), and astrocytes and Schwann cells within the brush border of the jejunum of the small intestine. PSMA has two major enzyme activities: hydrolytic cleavage of γ-biglutamic acid from poly γ-glutamyl folate, and proteolysis of the neuropeptide N-acetyl-L-aspartyl-L-glutamic acid (NAAG). In addition to having the enzymatic functions, PSMA is also upregulated (1000 times higher than the physiological level) and strongly expressed in prostate cancer cells, particularly in castration-resistant and metastatic prostate cancer, as well as in lymph node, bone, rectal, and lung metastatic tumor tissues. There are significant increases in PSMA expression in new blood vessels of tumor tissues, and its expression level is significantly correlated to the tumor's degree of differentiation, metastatic potential, and sensitivity to hormonal treatment. Research evidence shows that PSMA is highly expressed in almost all prostate cancer tissues. This makes PSMA a potential target that is highly sensitive and highly specific to metastatic foci of prostate cancer, which is an ideal biomarker; moreover, it can be used in targeted radionuclide therapy for the treatment of advanced cancer. Over the last few decades, the development of new radiopharmaceuticals targeting PSMA has mainly followed two different paths. Initially, studies focused mainly on the macromolecular protein structure of PSMA, providing specific monoclonal antibodies against PSMA epitopes. The first clinically used PSMA-targeting radiotracer was [¹¹¹In]capromab pendetide under the trademark ProstaScint^{™}, which was approved by the FDA as an imaging agent for PSMA in 1997. Capromab (7E11-C5) is a monoclonal antibody developed from the cell membrane of the human prostate cancer cell LNCaP and was labeled with indium-111 using diethylenetriaminepentaacetic acid (DTPA) as a chelating agent. Other second-generation drugs of PSMA-targeting monoclonal antibodies and antibody derivatives are currently under development, with J591 being the most extensively studied to date. Such a deimmunized monoclonal antibody has a high affinity for live cells that express PSMA on their membranes. It overcomes the main limitations of capromab, which can only target PSMA effectively through the disruption of the cell membrane and results in prolonged retention of radioactivity in non-target organs. By being coupled with the metal chelating agent, 1,4,7,10-tetraazacyclododecane-1,4,7,10-tetraacetic acid (DOTA), J591 has been successfully used in labeling, diagnosis and treatment with radioactive metal elements such as ¹¹¹In, ^{99m}Tc, ⁸⁹Zr, ⁹⁰Y, ¹⁷⁷Lu, and ²²⁵Ac. However, antibodies have serious limitations as a clinically conventional means of molecular imaging in that it takes a relatively long time (usually 3-7 days) to metabolize them *in vivo* to reduce the background of the blood circulation and thus to achieve an adequate signal-to-noise ratio; their size also limits their penetration of tumors.

As the crystal structure of PSMA has been resolved, various PSMA inhibitors with small molecular weight and the potential as radiopharmaceuticals have been synthesized and assessed based on the enzyme activities of PSMA. Small-molecular-weight ligands are easier to prepare on a large scale than antibodies and have good pharmacokinetic properties (such as bioavailability and biological half-life). Among them, glutamic acid-phosphoramides 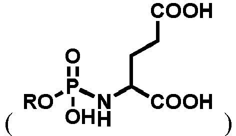 and glutamic acid-ureido derivatives 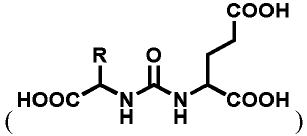 are two widely studied classes of chemical entities, and the use of the corresponding PSMA inhibitors in nuclear medicine has been studied. Among these compounds, ureido derivative inhibitors are the most commonly used PSMA-targeting molecules at present. In 2021, the FDA approved the use of [¹⁸F]DCFPyL in PET imaging of prostate cancer; it has not only a short residence time in the blood but also a high affinity to bind to PSMA and thus a relatively high tumor uptake. [¹⁸F]PSMA-1007, another F-18-labeled radioactive ligand, has good binding and internalization characteristics *in vitro* and has a relatively high specific uptake *in vivo.* In addition, compared to other known PSMA ligands, PSMA-1007 has a unique biodistribution and is almost exclusively excreted via the hepatobiliary route. This means that it is conducive to identifying metastatic recurrences of PCa in lymph nodes based on the radioactivity of urine or to identifying local recurrences in the bladder. The most widely used Ga-68-labeled PSMA-specific tracer is [⁶⁸Ga]PSMA-11 (also known as [⁶⁸Ga] Glu-Urea-Lys(Ahx)-HBED-CC), which structurally comprises a urea-based pharmacophore and a [⁶⁸Ga]HBED-CC complex and is capable of interacting directly with the hydrophobic binding pocket S1 of PSMA. [⁶⁸Ga]PSMA-11 is rapidly cleared from the blood and non-target organs, is lowly accumulated in the liver, and has a high specific uptake in organs and tumors that highly express PSMA. In addition, Benešová et al. reported the synthesis and preclinical evaluation of the ligand PSMA-617. PSMA-617 is a theranostic ligand. The chelating agent DOTA is coupled to the pharmacophore Glu-Urea-Lys through a naphthalene-containing linker. Lu-177-labeled PSMA-617 has a high binding affinity and internalization properties, a relatively high tumor uptake rate at a later point in time, and a relatively low accumulation in the spleen, and is cleared from the kidney with a relatively high efficiency.

Although there have been reports on the development of a large number of PSMA inhibitors, there remains a significant need for better PSMA-targeting drugs among prostate cancer patients. Therefore, developing a PSMA inhibitor that is stable *in vivo* and has higher affinity and specificity is of great scientific value, and such an inhibitor has a wide range of application prospects.

### SUMMARY

The present disclosure provides a compound represented by formula (I) or a pharmaceutically acceptable salt thereof, 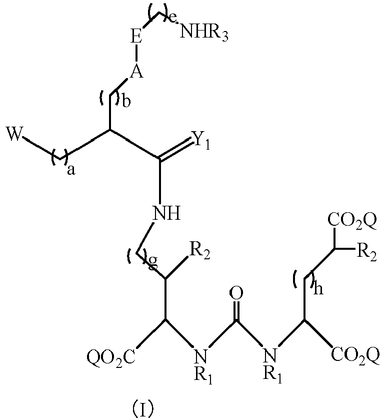 wherein:
Q is selected from the group consisting of H and a protecting group, preferably from H;
R₁ and R₂ are each independently selected from the group consisting of H and C₁₋₄ alkyl and are preferably both H; the C₁₋₄ alkyl is optionally substituted with one or more substituents P or is unsub stituted;
each occurrence of Q, R₁, and R₂ may be the same or different;
R₄ is selected from the group consisting of H, C₁-₆ alkyl, 6-10 membered aryl, and 5-12 membered heteroaryl; the C₁-₆ alkyl, 6-10 membered aryl, or 5-12 membered heteroaryl is optionally substituted with one or more substituents P or is unsubstituted;
Y₁ is S or O, preferably O;
A is selected from the group consisting of -NR₄(CO)-, -N R₄ (SO₂)-, -N R₄ (CH₂)-, and absent;
the E is selected from the group consisting of 3-12 membered cycloalkyl, 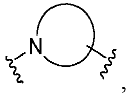 and absent, and the 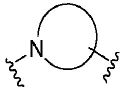 is heterocyclyl or heteroaryl comprising one or more N atoms, wherein the 3-12 membered cycloalkyl, heterocyclyl, or heteroaryl is optionally substituted with one or more substituents P or is unsubstituted;
when A is selected from the group consisting of -NR₄ (CO)- and absent, E is not cyclohexane;
W is selected from the group consisting of 3-12 membered cycloalkyl, 3-12 membered heterocycloalkyl, 6-10 membered aryl, and 5-12 membered heteroaryl; the C₃₋₁₂ cycloalkyl, 3-12 membered heterocycloalkyl, 6-10 membered aryl, or 5-12 membered heteroaryl is optionally substituted with one or more substituents P or is unsubstituted;
the substituents P are selected from the group consisting of C₁-C₆ alkyl, halogen, deuterium, hydroxy, sulfhydryl, -NRᵢRⱼ, oxo, thio, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, 5- to 10-membered heteroaryl, 8- to 12-membered fused cycloaryl, and 5- to 12-membered fused heteroaryl;
Rᵢ and Rⱼ are each independently selected from the group consisting of a hydrogen atom, hydroxy, C₁-C₆ alkyl, and C₁-C₆ alkoxy; Rₖ is independently selected from the group consisting of a hydrogen atom, C₁-C₆ alkyl, C₁-C₆ haloalkyl, C₁-C₆ alkoxy, hydroxy, and -NRᵢRⱼ, wherein the alkyl, alkoxy, or haloalkyl is optionally substituted with one or more substituents selected from the group consisting of C₁-C₆ alkyl, halogen, hydrogen, sulfhydryl, -NRᵢRⱼ, oxo, thio, carboxyl, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, and 5- to 10-membered heteroaryl;
a, b, e, g, and h are each independently integers of 0-6;
when A and E are both absent, W is not naphthyl;
R₃ is selected from the group consisting of H and a chelating agent.

In some embodiments, the A is -NH(CO)-, and E is selected from 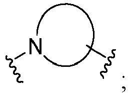 the 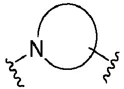 is heterocyclyl comprising one or more N atoms, and the heterocyclyl is preferably 3-12 membered heterocyclyl, more preferably a 3-8 membered monoheterocycle, and most preferably

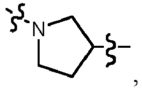

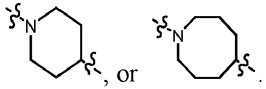

In some embodiments, the A is -NH(CO)-, and E is selected from 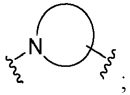 the 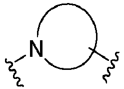 is heterocyclyl comprising one or more N atoms is 5-12 membered fused heterocyclyl, preferably

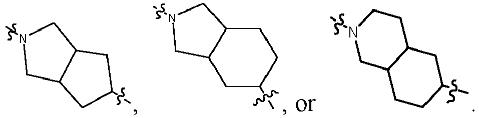

In some embodiments, the A is -NH(CO)-, and E is selected from 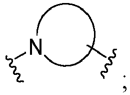 the 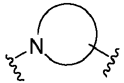 is heteroaryl comprising one or more N atoms is 5-12 membered fused heteroaryl, preferably

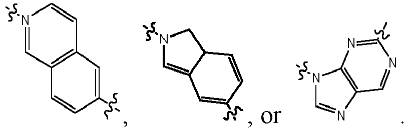

In some embodiments, the A is -NH(CO)-, and E is selected from 3-12 membered cycloalkyl and is not cyclohexane; the 3-12 membered cycloalkyl is 5-12 membered fused cycloalkyl, preferably

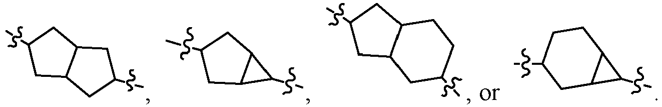

In some embodiments, the A is -NH(CO)-, e is 1, and E is selected from 3-12 membered cycloalkyl; the 3-12 membered cycloalkyl is spirocycloalkyl.

In some embodiments, the A is -NH(CO)-, e is 1, and E is selected from 3-12 membered cycloalkyl; the 3-12 membered cycloalkyl is 5-12 membered monospirocycloalkyl.

In some embodiments, the A is -NH(CO)-, e is 1, and E is selected from 3-12 membered cycloalkyl; the 3-12 membered cycloalkyl is 3-membered/4-membered, 3-membered/5-membered, 3-membered/6-membered, 4-membered/4-membered, 4-membered/5-membered, 4-membered/6-membered, 5-membered/5-membered, 5-membered/6-membered, or 6-membered/6-membered monospirocycloalkyl.

In some embodiments, the A is -NH(CO)-, e is 1, and E is selected from 3-12 membered cycloalkyl; the 3-12 membered cycloalkyl is selected from the group consisting of 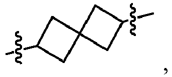 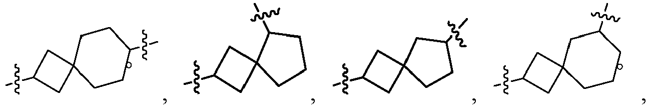 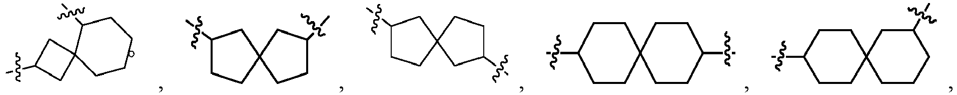 and

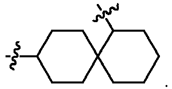

In some embodiments, the A is -NH(CO)-, e is 1, and E is selected from 3-12 membered cycloalkyl; the 3-12 membered cycloalkyl is

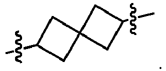

In some embodiments, the A is -NH(CO)-, and E is selected from 3-12 membered cycloalkyl; the 3-12 membered cycloalkyl is bridged cycloalkyl.

In some embodiments, the A is -NH(CO)-, and E is selected from 3-12 membered cycloalkyl is selected from the group consisting of

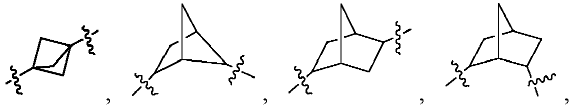

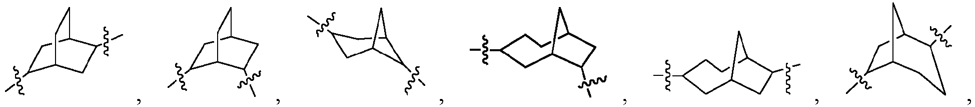

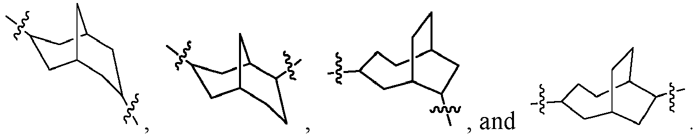

In some embodiments, the A is -NH(CO)-, and E is selected from 3-12 membered cycloalkyl is 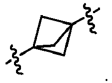 In some embodiments, the A is -NH(CO)-, and E is absent.

In some embodiments, in the compound represented by formula (I) or the pharmaceutically acceptable salt thereof provided by the present disclosure, the a is 1, and the W is selected from the group consisting of phenyl and naphthyl.

In some embodiments, in the compound represented by formula (I) or the pharmaceutically acceptable salt thereof provided by the present disclosure, the a is 1, and the W is naphthyl.

In some embodiments, in the compound represented by formula (I) or the pharmaceutically acceptable salt thereof provided by the present disclosure, the Q is selected from the group consisting of H and a protecting group.

In some embodiments, in the compound represented by formula (I) or the pharmaceutically acceptable salt thereof provided by the present disclosure, the Q is H.

In some embodiments, in the compound represented by formula (I) or the pharmaceutically acceptable salt thereof provided by the present disclosure, each R₂ is independently H.

In some embodiments, in the compound represented by formula (I) or the pharmaceutically acceptable salt thereof provided by the present disclosure, each R₁ is independently H.

In some embodiments, in the compound represented by formula (I) or the pharmaceutically acceptable salt thereof provided by the present disclosure, the h is selected from the group consisting of 1 and 2.

In some embodiments, in the compound represented by formula (I) or the pharmaceutically acceptable salt thereof provided by the present disclosure, the h is 1.

In some embodiments, in the compound represented by formula (I) or the pharmaceutically acceptable salt thereof provided by the present disclosure, the g is selected from the group consisting of 3 and 4.

In some embodiments, in the compound represented by formula (I) or the pharmaceutically acceptable salt thereof provided by the present disclosure, the g is 3.

In some embodiments, in the compound represented by formula (I) or the pharmaceutically acceptable salt thereof provided by the present disclosure, the a is 1, the W is naphthyl, and Q, R₂, and R₁ are each independently H.

In some embodiments, the compound of formula (I) is a compound represented by formula (I-1) or a pharmaceutically acceptable salt thereof, 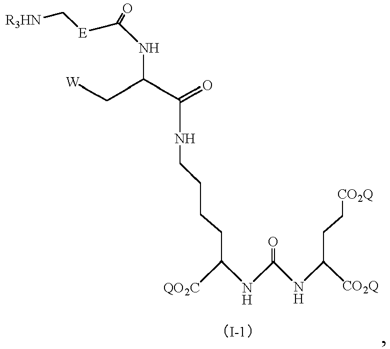 wherein:
Q is selected from the group consisting of H and a protecting group, preferably from H, and each occurrence may be the same or different;
E is selected from the group consisting of 3-12 membered cycloalkyl, 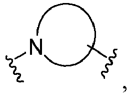 and absent, and the 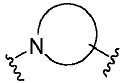 is heterocyclyl comprising one N atom, wherein the 3-12 membered cycloalkyl or heterocyclyl comprising one N atom is optionally substituted with one or more substituents P or is unsubstituted, and the 3-12 membered cycloalkyl is not cyclohexane; the 3-12 membered cycloalkyl is preferably fused cycloalkyl; the heterocyclyl comprising one N atom is preferably a 6-membered ring;
W is selected from 6-10 membered aryl, and the 6-10 membered aryl is optionally substituted with one or more substituents P or is unsubstituted;
the substituents P are selected from the group consisting of C₁-C₆ alkyl, halogen, deuterium, hydroxy, and sulfhydryl;
R₃ is selected from the group consisting of H and a chelating agent.

In some embodiments, in the compound represented by formula (I-1) or the pharmaceutically acceptable salt thereof, the W is phenyl or naphthyl.

In some embodiments, in the compound represented by formula (I) or the pharmaceutically acceptable salt thereof, the A is -NH(SO₂)-, and E is selected from C₃₋₁₂ cycloalkyl, preferably from 3-8 membered cycloalkyl, more preferably from cyclohexane, and most preferably from the group consisting of

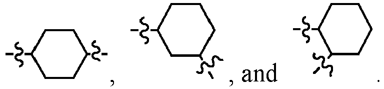

In some embodiments, in the compound represented by formula (I) or the pharmaceutically acceptable salt thereof, the A is -N(CH₂)-, and E is selected from 3-12 membered cycloalkyl, preferably from 3-12 membered cycloalkyl, more preferably from cyclohexane, and most preferably from the group consisting of

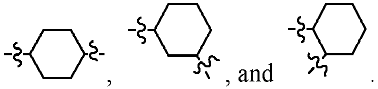

In some embodiments, the compound represented by formula (I) is a compound represented by formula (I-2) or a pharmaceutically acceptable salt thereof, 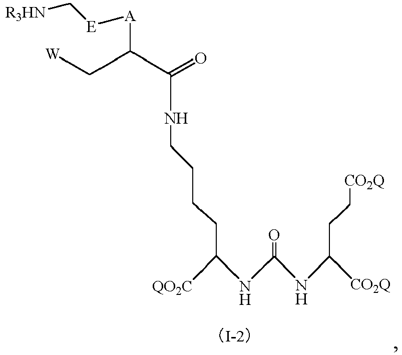 wherein:
Q is selected from the group consisting of H and a protecting group, preferably from H, and each occurrence may be the same or different;
A is selected from the group consisting of -NH(SO₂)- and -N(CH₂)-;
E is selected from the group consisting of 3-12 membered cycloalkyl and absent, wherein the 3-12 membered cycloalkyl is optionally substituted with one or more substituents P or is unsubstituted;
W is selected from 6-10 membered aryl, and the 6-10 membered aryl is optionally substituted with one or more substituents P or is unsubstituted;
the substituents P are selected from the group consisting of C₁-C₆ alkyl, halogen, deuterium, hydroxy, and sulfhydryl;
R₃ is selected from the group consisting of H and a chelating agent.

In some embodiments, in the compound represented by formula (I-2) or the pharmaceutically acceptable salt thereof, the W is naphthyl.

In some embodiments, in the compound represented by formula (I) or the pharmaceutically acceptable salt thereof, the A is absent, and E is selected from 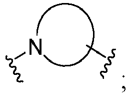 the 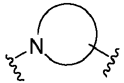 is 5-12 membered fused heterocyclyl comprising one or more N atoms, preferably

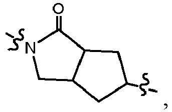

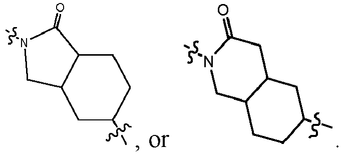

In some embodiments, in the compound represented by formula (I) or the pharmaceutically acceptable salt thereof, the A is absent, and E is selected from 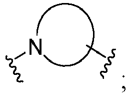 the 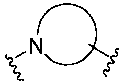 is 5-12 membered heteroaryl comprising one or more N atoms, preferably 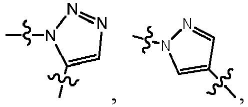 or

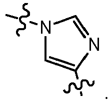

In some embodiments, the compound represented by formula (I) is a compound represented by formula (I-3) or a pharmaceutically acceptable salt thereof, 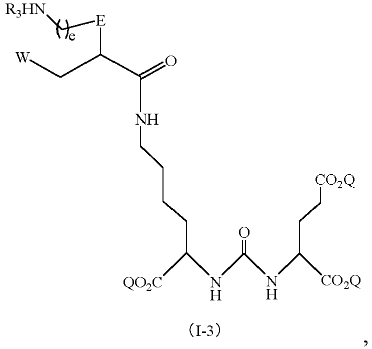 wherein:
Q is selected from the group consisting of H and a protecting group, preferably from H, and each occurrence may be the same or different;
E is selected from 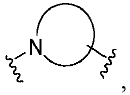 and the 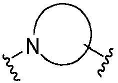 is 5-12 membered fused heterocyclyl or 5-12 membered heteroaryl comprising one or more N atoms; the 5-12 membered fused heterocyclyl or 5-12 membered heteroaryl comprising one or more N atoms is optionally substituted with one or more substituents P or is unsubstituted;
W is selected from 6-10 membered aryl, and the 6-10 membered aryl is optionally substituted with one or more substituents P or is unsubstituted;
the substituents P are selected from the group consisting of C₁-C₆ alkyl, halogen, deuterium, hydroxy, sulfhydryl, and carbonyl;
e is selected from the group consisting of 0 and 1;
R₃ is selected from the group consisting of H and a chelating agent.

In some embodiments, in the compound represented by formula (I-3) or the pharmaceutically acceptable salt thereof, the E is 5-12 membered heteroaryl comprising one or more N atoms and is unsubstituted, and is preferably

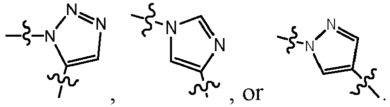

In some embodiments, in the compound represented by formula (I-3) or the pharmaceutically acceptable salt thereof, the E is 5-12 membered fused heterocyclyl comprising one or more N atoms, and the 5-12 membered fused heterocyclyl comprising one N atom is substituted with carbonyl and is preferably

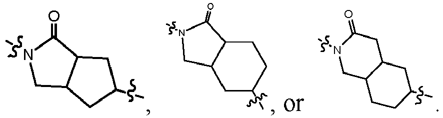

In some embodiments, in the compound represented by formula (I-3) or the pharmaceutically acceptable salt thereof, the W is phenyl or naphthyl.

In some embodiments, in the compound represented by formula (I) or the pharmaceutically acceptable salt thereof, the A and E are both absent.

In some embodiments, the compound represented by formula (I) is a compound represented by formula (I-4) or a pharmaceutically acceptable salt thereof, 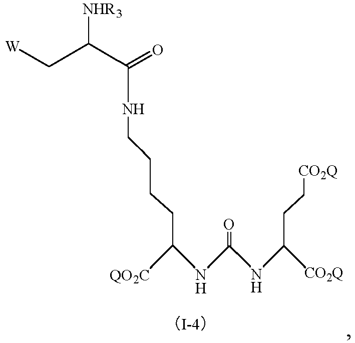 wherein:
Q is selected from the group consisting of H and a protecting group, preferably from H, and each occurrence may be the same or different;
W is selected from the group consisting of 6-10 membered aryl and 5-12 membered heteroaryl; the 6-10 membered aryl or 5-12 membered heteroaryl is optionally substituted with one or more substituents P or is unsubstituted;
the substituents P are selected from the group consisting of C₁-C₆ alkyl, halogen, deuterium, hydroxy, and sulfhydryl;
R₃ is selected from the group consisting of H and a chelating agent.

In some embodiments, in the compound represented by formula (I-4) or the pharmaceutically acceptable salt thereof, the W is selected from 6-10 membered aryl, preferably from the group consisting of phenyl, naphthyl, biphenyl, and phenylhydroxy, and more preferably from the group consisting of phenyl,

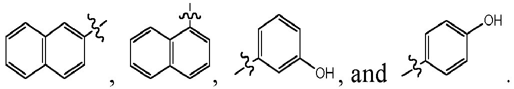

In some embodiments, in the compound represented by formula (I-4) or the pharmaceutically acceptable salt thereof, the W is selected from 5-12 membered heteroaryl, preferably from 5-6 membered heteroaryl or fused heteroaryl, more preferably from the group consisting of indole, pyridine, imidazole, and quinoline, and most preferably from the group consisting of

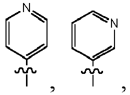

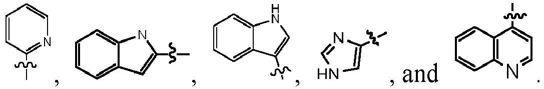

The present disclosure further provides a compound represented by formula (II) or a pharmaceutically acceptable salt thereof, 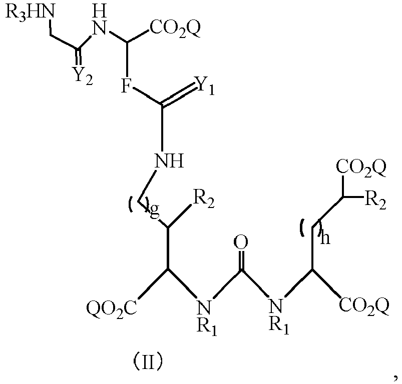 wherein:
Q is selected from the group consisting of H and a protecting group, preferably from H;
R₁ and R₂ are independently selected from the group consisting of H and substituted or unsubstituted C₁₋₄ alkyl and are preferably both H;
each occurrence of Q, R₁, and R₂ may be the same or different;
F is selected from the group consisting of -N(CH₂)ₙ- and -(CH₂)ₘOG(CH₂)ₙ-;
Y₁ and Y₂ are independently selected from the group consisting of S and O, preferably from O;
g, h, n, and m are each independently selected from the group consisting of integers of 0-6;
G is selected from the group consisting of 3-12 membered cycloalkyl, 3-12 membered heterocycloalkyl, 6-10 membered aryl, and 5-12 membered heteroaryl; the 3-12 membered cycloalkyl, 3-12 membered heterocycloalkyl, 6-10 membered aryl, or 5-12 membered heteroaryl is optionally substituted with one or more substituents P or is unsubstituted;
the substituents P are selected from the group consisting of C₁-C₆ alkyl, halogen, deuterium, hydroxy, sulfhydryl, -NRᵢRⱼ, oxo, thio, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, 5- to 10-membered heteroaryl, 8- to 12-membered fused cycloaryl, and 5- to 12-membered fused heteroaryl;
Rᵢ and Rⱼ are each independently selected from the group consisting of a hydrogen atom, hydroxy, C₁-C₆ alkyl, and C₁-C₆ alkoxy; Rₖ is independently selected from the group consisting of a hydrogen atom, C₁-C₆ alkyl, C₁-C₆ haloalkyl, C₁-C₆ alkoxy, hydroxy, and -NRᵢRⱼ, wherein the alkyl, alkoxy, or haloalkyl is optionally substituted with one or more substituents selected from the group consisting of C₁-C₆ alkyl, halogen, hydrogen, sulfhydryl, -NRᵢRⱼ, oxo, thio, carboxyl, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, and 5- to 10-membered heteroaryl;
R₃ is selected from the group consisting of H and a chelating agent.

In some embodiments, in the compound represented by formula (II) or the pharmaceutically acceptable salt thereof, the F is selected from -N(CH₂)ₙ-.

In some embodiments, in the compound represented by formula (II) or the pharmaceutically acceptable salt thereof, the F is selected from -(CH₂)ₘOG(CH₂)ₙ-, and the G is selected from 6-10 membered aryl, preferably from phenyl.

In some embodiments, the compound represented by formula (II) is a compound represented by formula (II-1) or a pharmaceutically acceptable salt thereof, 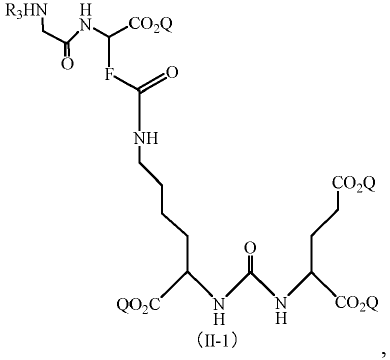 wherein:
Q is selected from the group consisting of H and a protecting group, preferably from H, and each occurrence may be the same or different;
F is selected from the group consisting of -N(CH₂)ₙ- and -(CH₂)ₘOG(CH₂)ₙ-;
the G is selected from 6-10 membered aryl;
n and m are each independently selected from the group consisting of integers of 0-6;
R₃ is selected from the group consisting of H and a chelating agent.

In some embodiments, in the compound represented by formula (II-1), the G is phenyl.

The present disclosure further provides a compound represented by formula (III) or a pharmaceutically acceptable salt thereof, 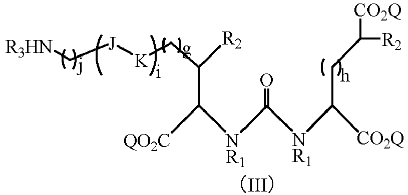 wherein:
Q is selected from the group consisting of H and a protecting group, preferably from H;
R₁ and R₂ are independently selected from the group consisting of H and substituted or unsubstituted C₁₋₄ alkyl and are preferably both H;
each occurrence of Q, R₁, and R₂ may be the same or different;
g, h, and j are each independently selected from the group consisting of integers of 0-6;
i is selected from the group consisting of integers of 1-3;
J is a linking group selected from the group consisting of C₁-C₆ alkylene, C₃-C₆ cycloalkylene, arylene, and heteroarylene; the C₁-C₆ alkylene, C₃-C₆ cycloalkylene, arylene, and heteroarylene are optionally substituted with one or more substituents P or are unsubstituted;
the substituents P are selected from the group consisting of C₁-C₆ alkyl, halogen, deuterium, hydroxy, sulfhydryl, -NRᵢRⱼ, oxo, thio, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, 5- to 10-membered heteroaryl, 8- to 12-membered fused cycloaryl, and 5- to 12-membered fused heteroaryl;
Rᵢ and Rⱼ are each independently selected from the group consisting of a hydrogen atom, hydroxy, C₁-C₆ alkyl, and C₁-C₆ alkoxy; Rₖ is independently selected from the group consisting of a hydrogen atom, C₁-C₆ alkyl, C₁-C₆ haloalkyl, C₁-C₆ alkoxy, hydroxy, and -NRᵢRⱼ, wherein the alkyl, alkoxy, or haloalkyl is optionally substituted with one or more substituents selected from the group consisting of C₁-C₆ alkyl, halogen, hydrogen, sulfhydryl, -NRᵢRⱼ, oxo, thio, carboxyl, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, and 5- to 10-membered heteroaryl;
K is selected from the group consisting of -NR5-(C=O)-, -NR5-(C=S)-, -(C=O)-NR5-, and -(C=S)-NR5-;
the R₅ is selected from the group consisting of H and C₁-C₄ alkyl;
R₃ is selected from the group consisting of H and a chelating agent.

In some embodiments, in the compound represented by formula (III) or the pharmaceutically acceptable salt thereof, i is selected from 2, K is selected from -NH-(C=O)-, and J is selected from the group consisting of arylene and heteroarylene, preferably from the group consisting of pyridine and phenyl, and more preferably from the group consisting of

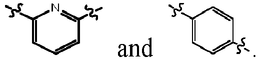

In some embodiments, the compound represented by formula (III) is a compound represented by formula (III-1) or a pharmaceutically acceptable salt thereof, 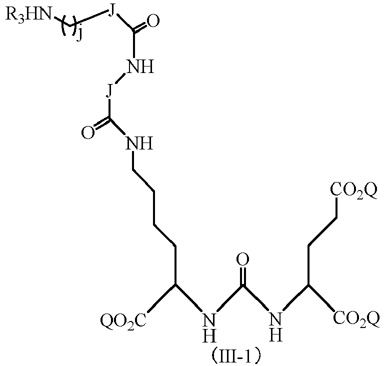 wherein:
Q is selected from the group consisting of H and a protecting group, preferably from H;
J is a linking group selected from the group consisting of arylene and heteroarylene; the arylene and heteroarylene are optionally substituted with one or more substituents P or are unsubstituted;
the substituents P are selected from the group consisting of C₁-C₆ alkyl, halogen, deuterium, hydroxy, sulfhydryl, oxo, and thio;
wherein each occurrence of Q and J may be the same or different;
j is selected from the group consisting of integers of 0-6;
R₃ is selected from the group consisting of H and a chelating agent.

In some embodiments, in the compound represented by formula (III) or the pharmaceutically acceptable salt thereof, J is selected from the group consisting of pyridine and phenyl, preferably from the group consisting of 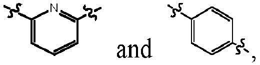 wherein each occurrence of J is different, and j is selected from 1.

The present disclosure provides a compound represented by formula (IV) or a pharmaceutically acceptable salt thereof, 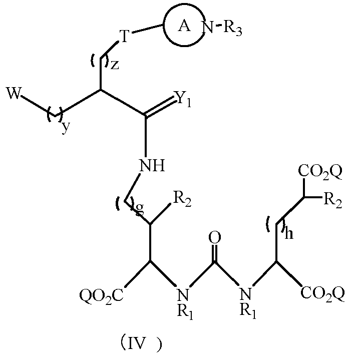 wherein:
Q is selected from the group consisting of H and a protecting group;
R₁ and R₂ are each independently selected from the group consisting of H and C₁₋₄ alkyl; the C₁₋₄ alkyl is optionally substituted with one or more substituents P or is unsubstituted;
each occurrence of Q, R₁, and R₂ may be the same or different;
Y₁ is S or O;
T is selected from the group consisting of -NR₄(CO)-, -NR₄ (SO₂)-, and -N R₄ (CH₂)-;
R₄ is selected from the group consisting of H, C₁-₆ alkyl, 6-10 membered aryl, and 5-12 membered heteroaryl; the C₁-₆ alkyl, 6-10 membered aryl, or 5-12 membered heteroaryl is optionally substituted with one or more substituents P or is unsubstituted;
ring A is selected from 3-12 membered nitrogen-containing heterocyclyl, wherein the 3-12 membered nitrogen-containing heterocyclyl is optionally substituted with one or more substituents P or is unsubstituted;
W is selected from the group consisting of 6-10 membered aryl and 5-12 membered heteroaryl; the -10 membered aryl or 5-12 membered heteroaryl is optionally substituted with one or more substituents P or is unsubstituted;
the substituents P are selected from the group consisting of C₁-C₆ alkyl, halogen, deuterium, hydroxy, sulfhydryl, -NRᵢRⱼ, oxo, thio, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, 5- to 10-membered heteroaryl, 8- to 12-membered fused cycloaryl, and 5- to 12-membered fused heteroaryl;
Rᵢ and Rⱼ are each independently selected from the group consisting of a hydrogen atom, hydroxy, C₁-C₆ alkyl, and C₁-C₆ alkoxy; Rₖ is independently selected from the group consisting of a hydrogen atom, C₁-C₆ alkyl, C₁-C₆ haloalkyl, C₁-C₆ alkoxy, hydroxy, and -NRᵢRⱼ, wherein the alkyl, alkoxy, or haloalkyl is optionally substituted with one or more substituents selected from the group consisting of C₁-C₆ alkyl, halogen, hydrogen, sulfhydryl, -NRᵢRⱼ, oxo, thio, carboxyl, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, and 5- to 10-membered heteroaryl;
y, z, g, and h are each independently integers of 0-6;
R₃ is selected from the group consisting of H and a chelating agent.

In some embodiments, in the compound represented by formula (IV) or the pharmaceutically acceptable salt thereof, Q is a protecting group; specifically, Q may be a hydroxy-protecting group; the hydroxy-protecting group includes all groups that can be generally used as protecting groups for hydroxy groups, and examples include the groups described in W. Greene et al., Protective Groups in Organic Synthesis, 4th edition, pp. 16-366, 2007, John Wiley & Sons, INC. Specific examples include C₁₋₆ alkyl, C₂₋₆ alkenyl, aryl C₁₋₆ alkyl group, C₁₋₆ alkoxy C₁₋₆ alkyl, acyl, C₁₋₆ alkoxycarbonyl, C₁₋₆ alkylsulfonyl, arylsulfonyl, tetrahydrofuranyl, tetrahydropyranyl, silyl, or the like.

In some embodiments, in the compound represented by formula (IV) or the pharmaceutically acceptable salt thereof provided by the present disclosure, the T is -NH(CO)-, and ring A is 5-12 membered nitrogen-containing spiroheterocyclyl.

In some embodiments, in the compound represented by formula (IV) or the pharmaceutically acceptable salt thereof provided by the present disclosure, the T is -NH(CO)-, and ring A is selected from 5-12 membered nitrogen-containing monospiroheterocyclyl.

In some embodiments, in the compound represented by formula (IV) or the pharmaceutically acceptable salt thereof provided by the present disclosure, the T is -NH(CO)-, and ring A is selected from the group consisting of 3-membered/4-membered, 3-membered/5-membered, 3-membered/6-membered, 4-membered/4-membered, 4-membered/5-membered, 4-membered/6-membered, 5-membered/5-membered, 5-membered/6-membered, and 6-membered/6-membered nitrogen-containing monospiroheterocyclyl groups.

In some embodiments, in the compound represented by formula (IV) or the pharmaceutically acceptable salt thereof provided by the present disclosure, the T is -NH(CO)-, and ring A is selected from the group consisting of

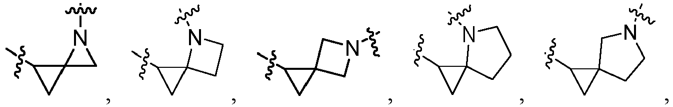

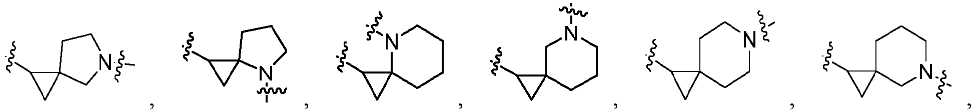

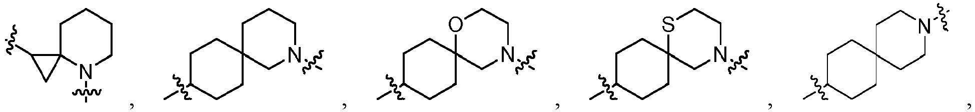

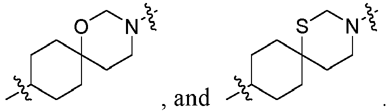

In some embodiments, in the compound represented by formula (IV) or the pharmaceutically acceptable salt thereof provided by the present disclosure, the T is -NH(CO)-, and ring A is selected from the group consisting of

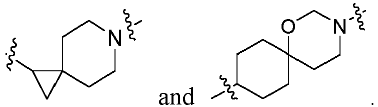

In some embodiments, in the compound represented by formula (IV) or the pharmaceutically acceptable salt thereof provided by the present disclosure, the T is -NH(CO)-, and ring A is

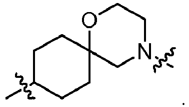

In some embodiments, in the compound represented by formula (IV) or the pharmaceutically acceptable salt thereof provided by the present disclosure, the W is selected from 6-10 membered aryl.

In some embodiments, in the compound represented by formula (IV) or the pharmaceutically acceptable salt thereof provided by the present disclosure, the W is naphthyl.

In some embodiments, in the compound represented by formula (IV) or the pharmaceutically acceptable salt thereof provided by the present disclosure, the Y₁ is O.

In some embodiments, in the compound represented by formula (IV) or the pharmaceutically acceptable salt thereof provided by the present disclosure, the R₁ and R₂ are each independently H.

In some embodiments, in the compound represented by formula (IV) or the pharmaceutically acceptable salt thereof provided by the present disclosure, the Q is selected from the group consisting of H and a protecting group.

In some embodiments, in the compound represented by formula (IV) or the pharmaceutically acceptable salt thereof provided by the present disclosure, the Q is H.

In some embodiments, in the compound represented by formula (IV) or the pharmaceutically acceptable salt thereof provided by the present disclosure, the y and h are each independently selected from the group consisting of 0, 1, and 2.

In some embodiments, in the compound represented by formula (IV) or the pharmaceutically acceptable salt thereof provided by the present disclosure, the y is 1.

In some embodiments, in the compound represented by formula (IV) or the pharmaceutically acceptable salt thereof provided by the present disclosure, the h is 1.

In some embodiments, in the compound represented by formula (IV) or the pharmaceutically acceptable salt thereof provided by the present disclosure, the g is selected from the group consisting of 3 and 4.

In some embodiments, in the compound represented by formula (IV) or the pharmaceutically acceptable salt thereof provided by the present disclosure, the g is 3.

In some embodiments, in the compound represented by formula (IV) or the pharmaceutically acceptable salt thereof provided by the present disclosure, the z is selected from the group consisting of 0 and 1.

In some embodiments, in the compound represented by formula (IV) or the pharmaceutically acceptable salt thereof provided by the present disclosure, the z is 0.

In some embodiments, the aforementioned chelating agent is selected from the group consisting of: 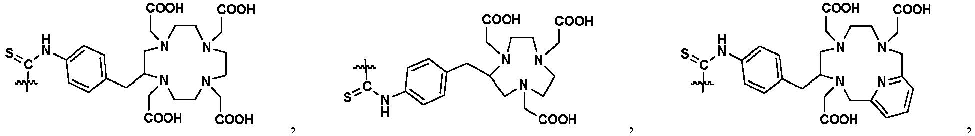 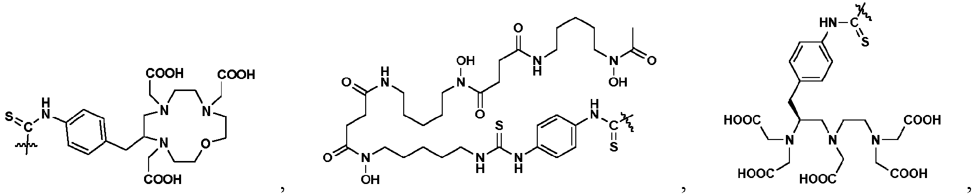 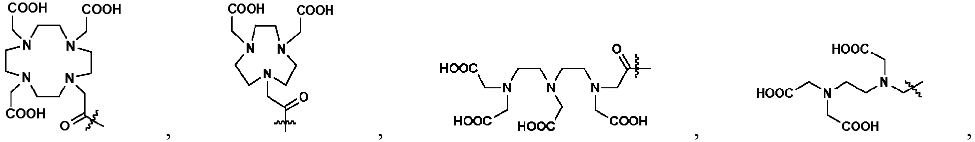 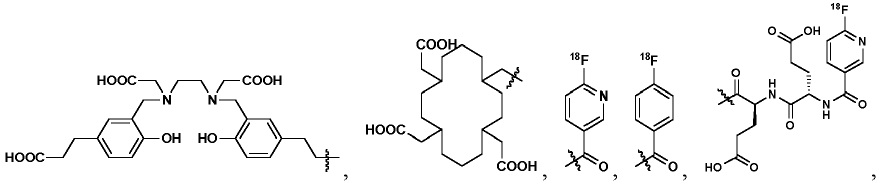 and

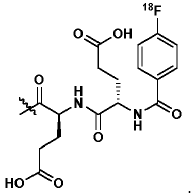

In some embodiments, the aforementioned chelating agent is

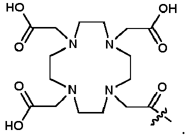

In some embodiments, the aforementioned chelating agent is

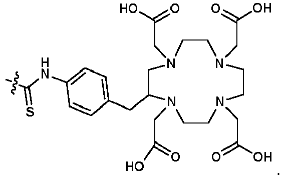

In some embodiments, the aforementioned compounds are selected from Table 1 below:

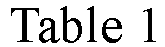

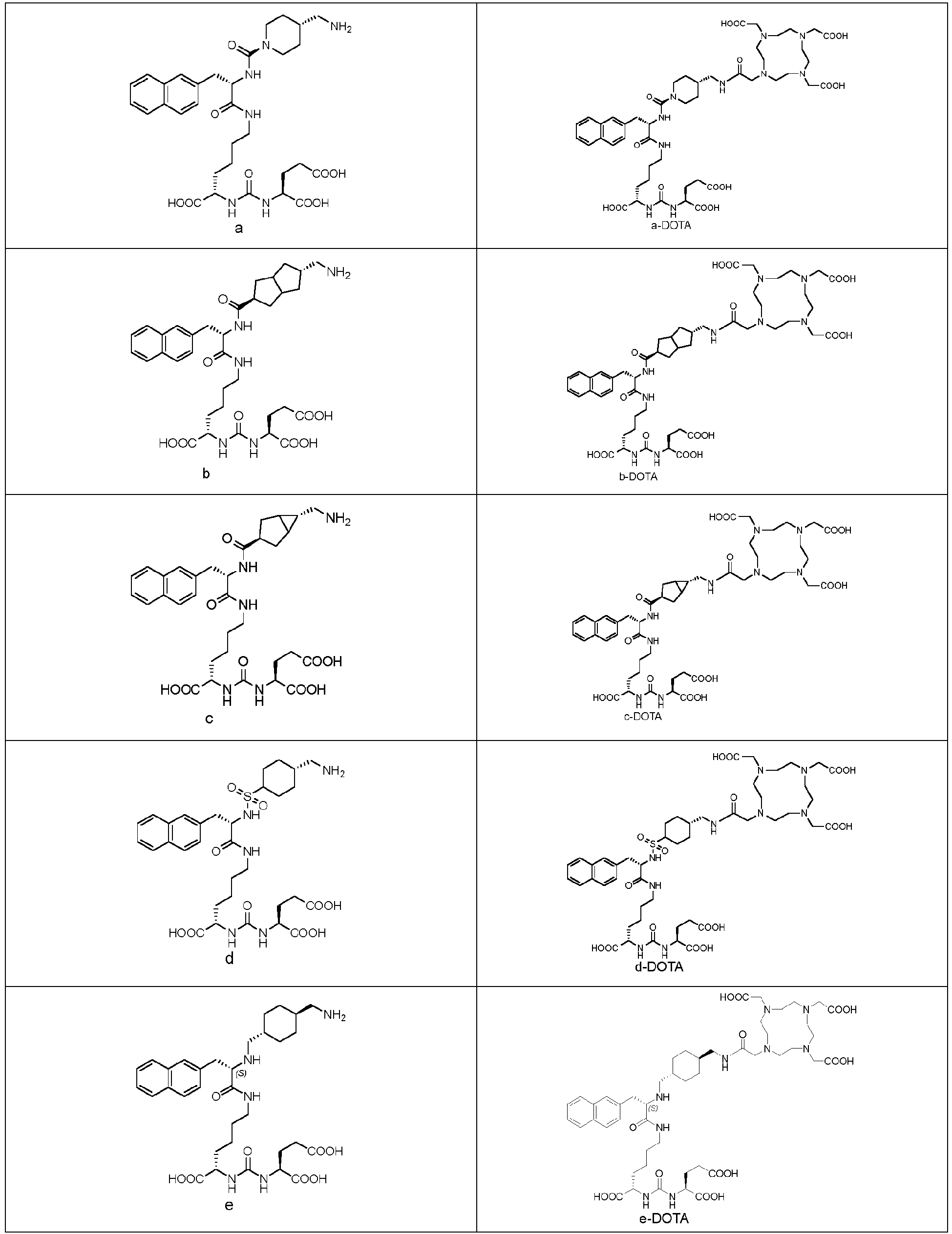

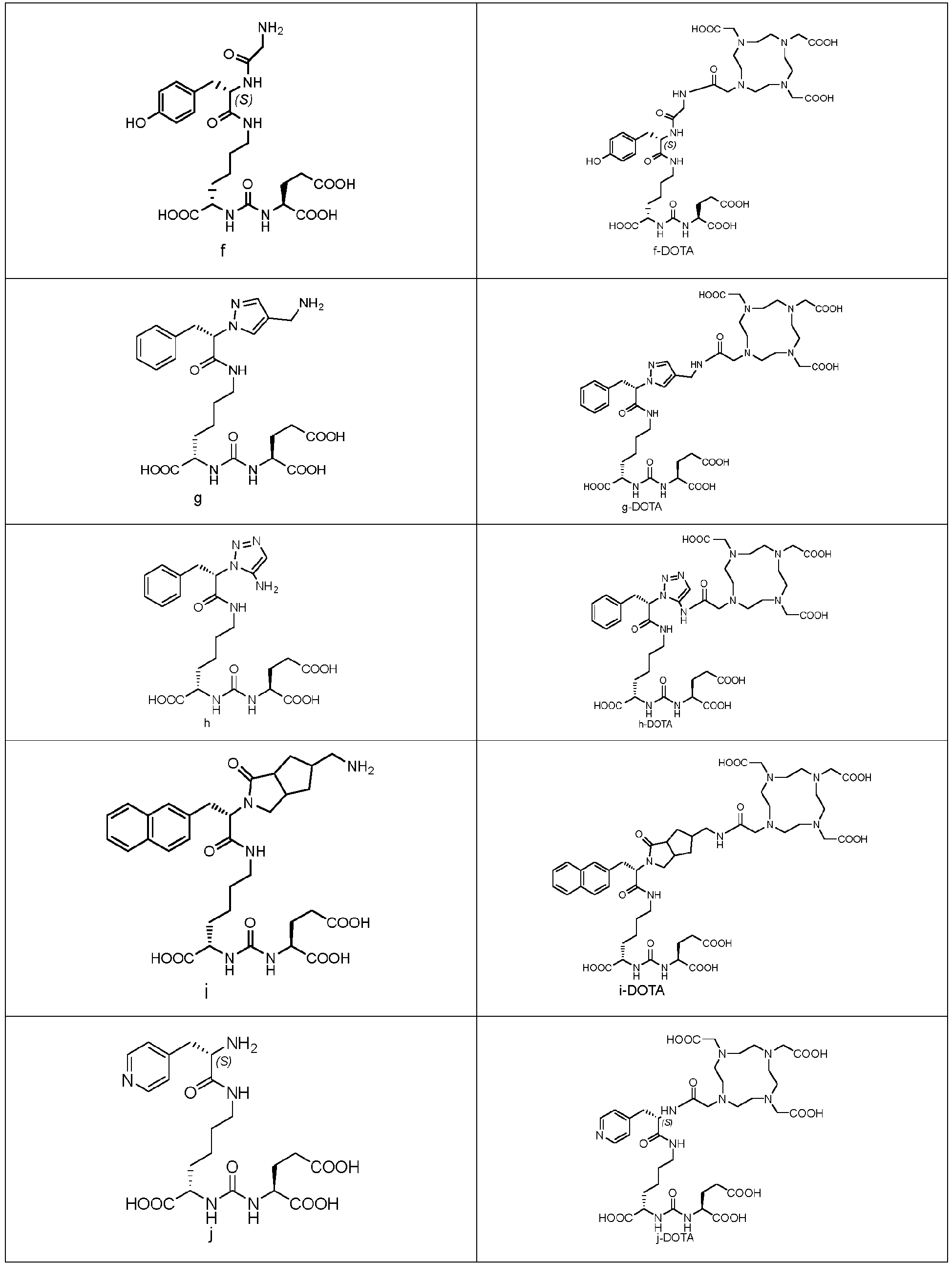

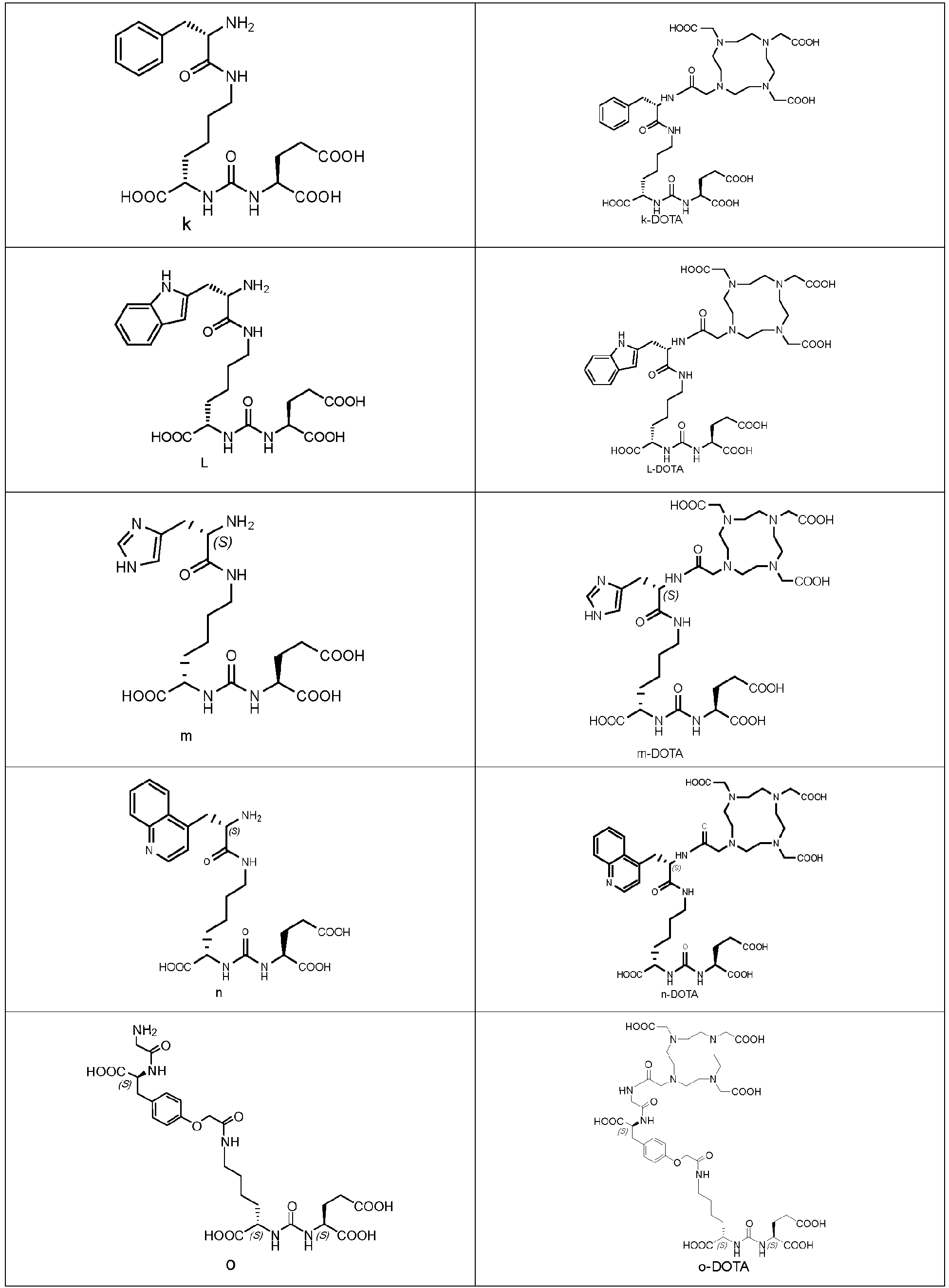

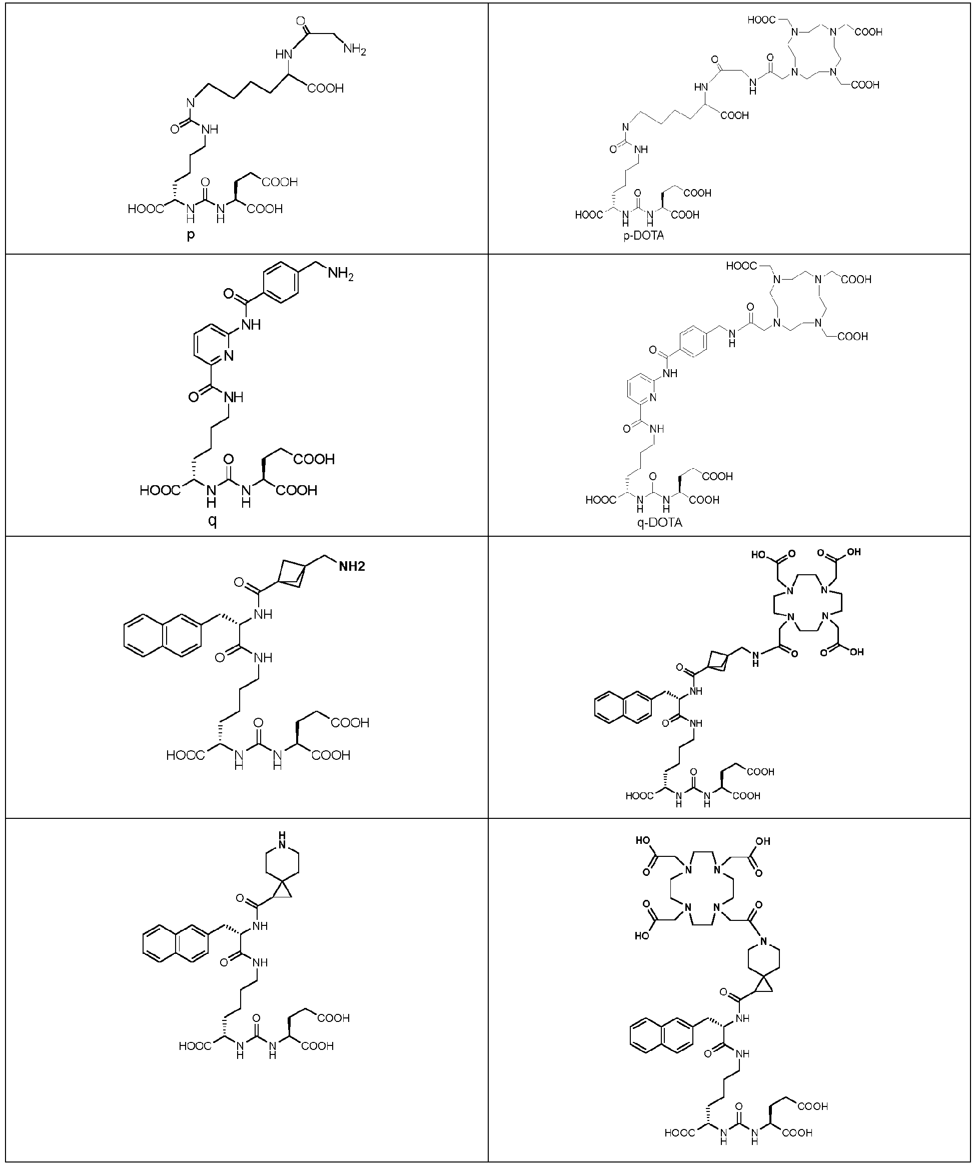

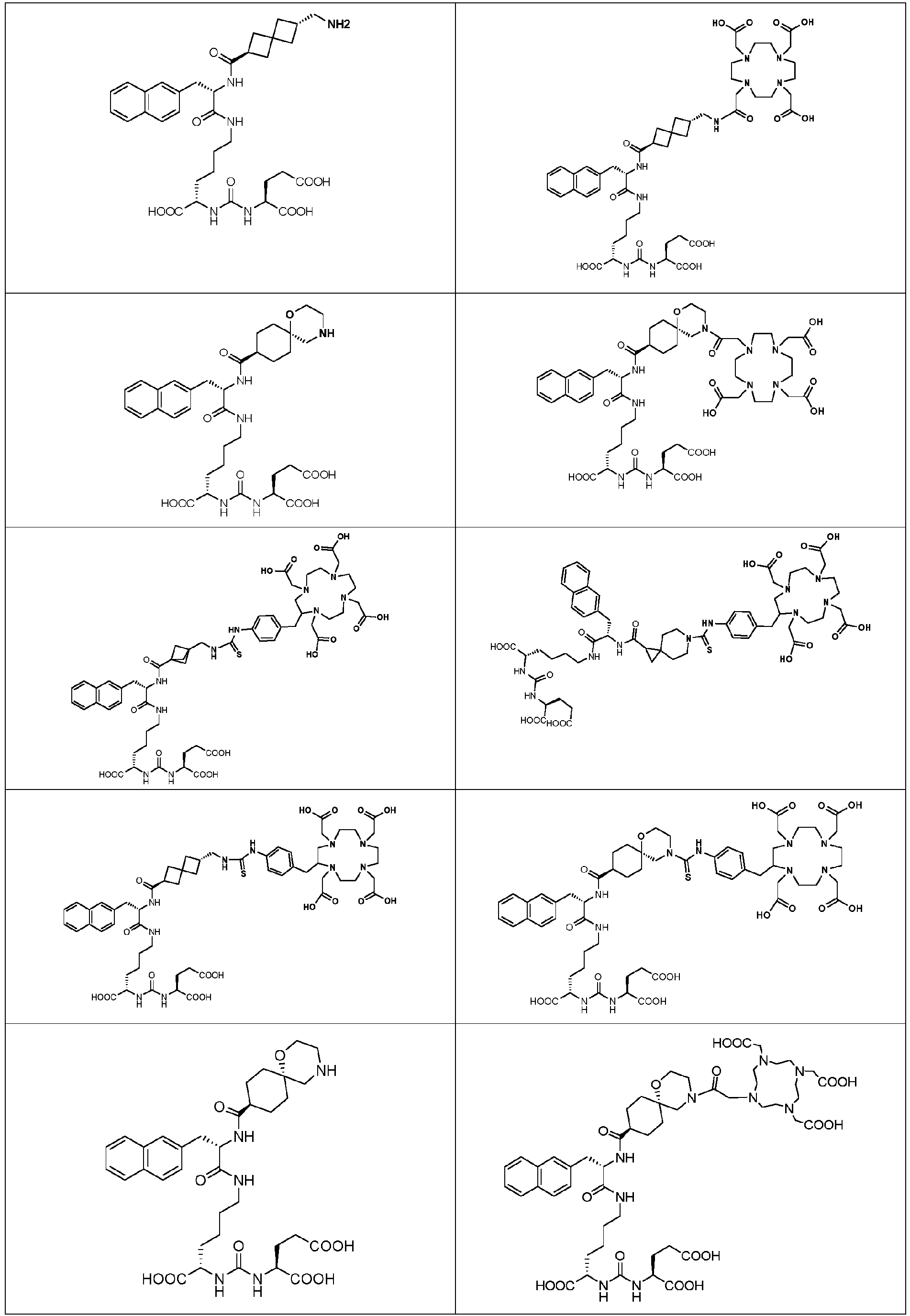

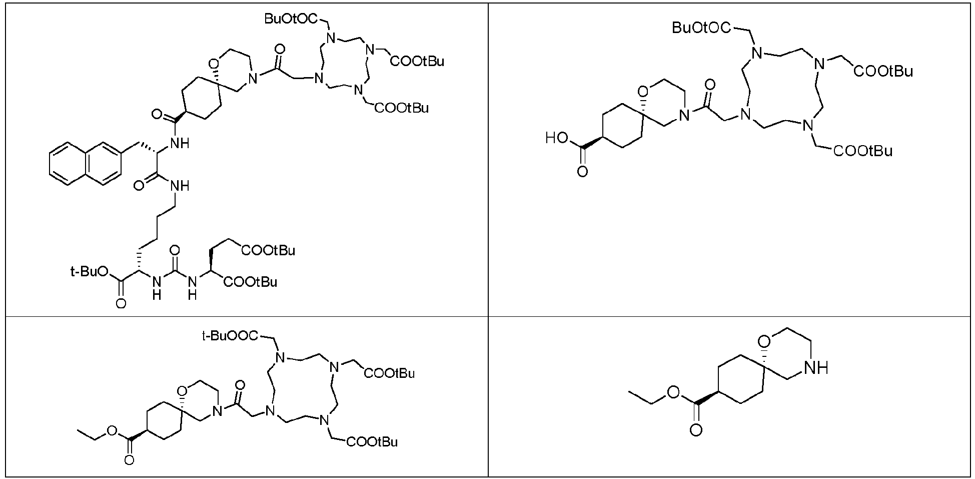

In some embodiments, the aforementioned chelating agent of the present disclosure comprises a radionuclide.

In some embodiments, the aforementioned radionuclide is selected from at least one of ¹⁸F, ¹¹C, ⁶⁸Ga , ¹²⁴I, ⁸⁹Zr, ⁶⁴Cu, ⁸⁶Y, ^{99m}Tc , ¹¹¹In, ¹²³I, ⁹⁰Y, ¹²⁵I, ¹³¹I, ¹⁷⁷Lu, ²¹¹At, ¹⁵³Sm, ¹⁸⁶Re, ¹⁸⁸Re, ⁶⁷Cu, ²¹²Pb, ²²⁵Ac, ²¹³Bi, ²¹²Bi, ²¹²Pb, and ⁶⁷Ga.

In some embodiments, the aforementioned radionuclide is ⁶⁸Ga.

In some embodiments, the aforementioned radionuclide is ¹⁷⁷Lu.

In some embodiments, the present disclosure provides a compound represented by formula (IV) or a pharmaceutically acceptable salt thereof, which is

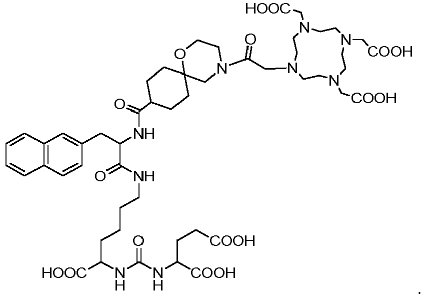

In an alternative embodiment, the compound represented by formula (IV) or the pharmaceutically acceptable salt thereof provided by the present disclosure is

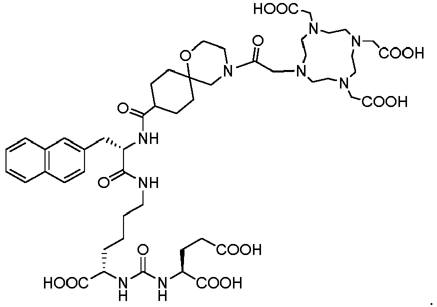

In some embodiments, the present disclosure provides a compound represented by formula (IV) or a pharmaceutically acceptable salt thereof, which is 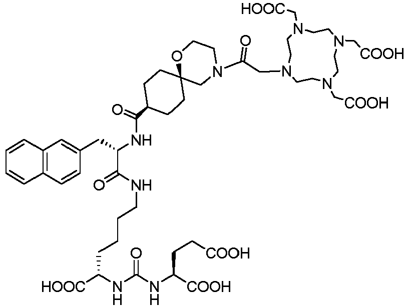 or

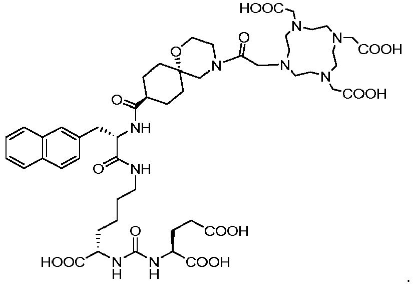

In some embodiments, the present disclosure provides a compound represented by formula (IV) or a pharmaceutically acceptable salt thereof, which is

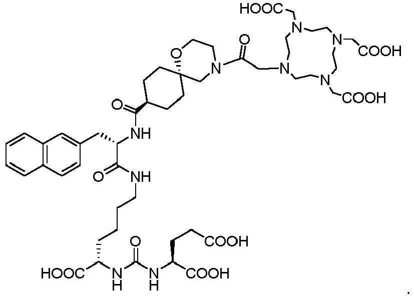

In some embodiments, the present disclosure provides a compound represented by formula (IV) or a pharmaceutically acceptable salt thereof, which is 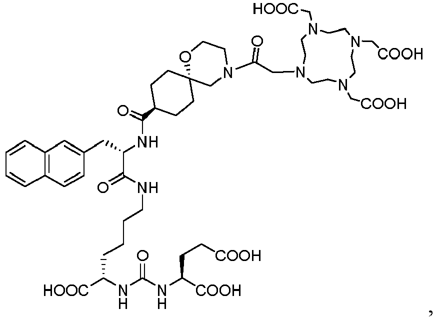 wherein the chelating agent comprises a radionuclide, and the radionuclide is ⁶⁸Ga.

In some embodiments, the present disclosure provides a compound represented by formula (IV) or a pharmaceutically acceptable salt thereof, which is 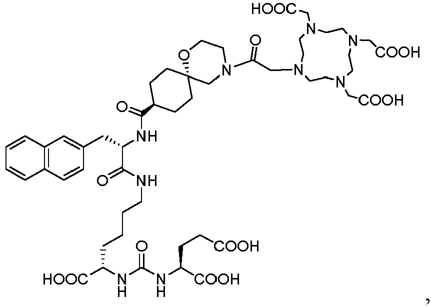 wherein the chelating agent comprises a radionuclide, and the radionuclide is ¹⁷⁷Lu.

The present disclosure further provides a preparation method for a compound represented by formula (IV) or a pharmaceutically acceptable salt thereof, wherein the compound represented by formula (IV) is the compound represented by formula v or a pharmaceutically acceptable salt thereof; the preparation method comprises the step of removing tert-butyl groups from the compound represented by formula v-5:

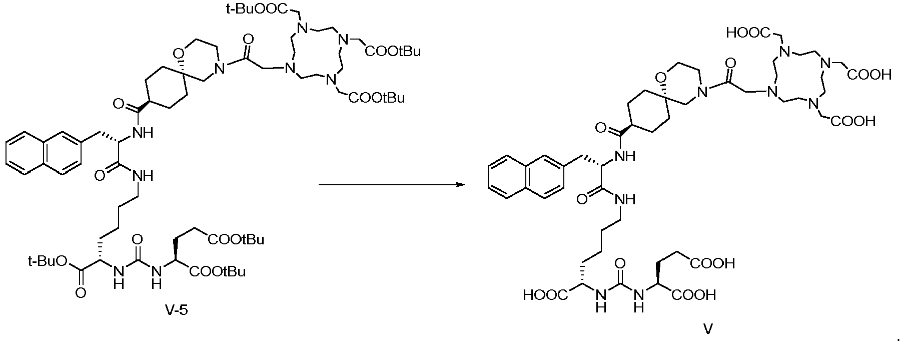

In an alternative embodiment, the preparation method for a compound represented by formula (IV) or a pharmaceutically acceptable salt thereof further comprises the step of conducting a condensation reaction of the compound represented by formula v-3 with the compound represented by formula v-4 to give the compound represented by formula v-5,

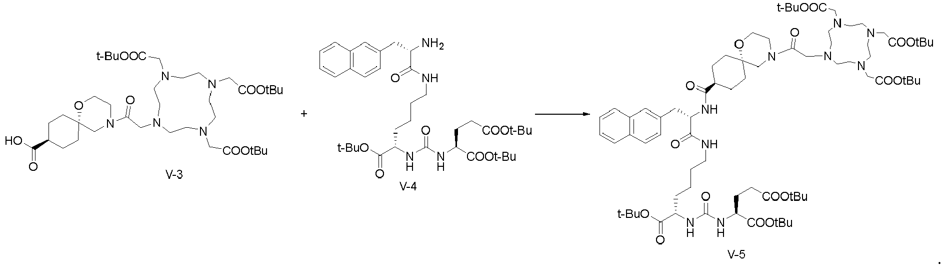

The preparation method for a compound represented by formula (IV) or a pharmaceutically acceptable salt thereof comprises the step of preparing the compound represented by formula (IV) or the pharmaceutically acceptable salt thereof and further comprises the step of complexing the chelating agent in the compound represented by formula (IV) or the pharmaceutically acceptable salt thereof with the radionuclide.

In an alternative embodiment, the present disclosure provides a preparation method for a compound represented by formula (IV) or a pharmaceutically acceptable salt thereof, wherein
the compound represented by formula (IV) or the pharmaceutically acceptable salt thereof is 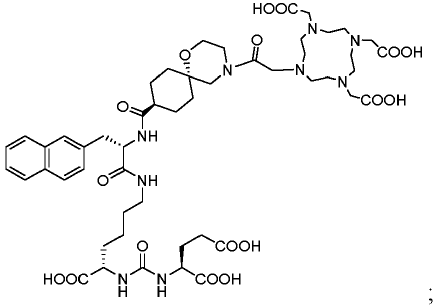
the preparation method comprises the step of obtaining individual isomers by conventional resolution of

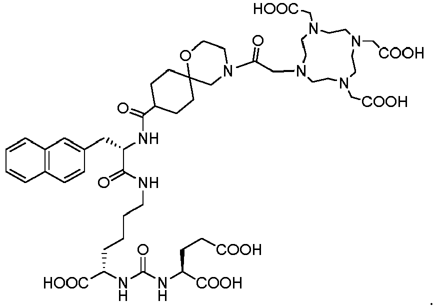

In some embodiments, the compound may be labeled with the aforementioned radionuclide.

The present disclosure further provides an isotopically substituted form of the aforementioned compound, preferably a deuterated compound.

The present disclosure further provides a pharmaceutical composition, comprising at least one of the aforementioned compounds or the pharmaceutically acceptable salts thereof, and a pharmaceutically acceptable carrier, diluent, or excipient.

In certain embodiments, a unit dose of the pharmaceutical composition is 0.001 mg-1000 mg.

In certain embodiments, the pharmaceutical composition comprises 0.01%-99.99% of the aforementioned compound based on the total weight of the composition. In certain embodiments, the pharmaceutical composition comprises 0.1%-99.9% of the aforementioned compound. In certain embodiments, the pharmaceutical composition comprises 0.5%-99.5% of the aforementioned compound. In certain embodiments, the pharmaceutical composition comprises 1%-99% of the aforementioned compound. In certain embodiments, the pharmaceutical composition comprises 2%-98% of the aforementioned compound.

In certain embodiments, the pharmaceutical composition comprises 0.01%-99.99% of the pharmaceutically acceptable carrier, diluent, or excipient based on the total weight of the composition. In certain embodiments, the pharmaceutical composition comprises 0.1%-99.9% of the pharmaceutically acceptable carrier, diluent, or excipient. In certain embodiments, the pharmaceutical composition comprises 0.5%-99.5% of the pharmaceutically acceptable carrier, diluent, or excipient. In certain embodiments, the pharmaceutical composition comprises 1%-99% of the pharmaceutically acceptable carrier, diluent, or excipient. In certain embodiments, the pharmaceutical composition comprises 2%-98% of the pharmaceutically acceptable carrier, diluent, or excipient.

The present disclosure further provides use of the aforementioned compound or the pharmaceutically acceptable salt thereof and the isotopically substituted form thereof in the preparation of a composition for imaging in patients.

The present disclosure further provides use of the aforementioned compound or the pharmaceutically acceptable salt thereof and the isotopically substituted form thereof in the preparation of a medicament for diagnosing and/or treating and/or preventing PSMA-mediated diseases or disorders.

The present disclosure further provides use of the aforementioned compound or the pharmaceutically acceptable salt thereof and the isotopically substituted form thereof in the preparation of a medicament for diagnosing and/or treating and/or preventing a tumor and a cancer, wherein the tumor and the cancer is preferably prostate cancer and/or a metastasis thereof.

### Terms and definitions:

Unless otherwise stated, the terms used in the specification and claims have the following meanings.

The term "alkyl" refers to a saturated aliphatic hydrocarbon group which is a linear or branched group containing 1 to 20 carbon atoms, preferably an alkyl group containing 1 to 12 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, and the like. More preferred is an alkyl group containing 1 to 6 carbon atoms; non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. Alkyl may be substituted or unsubstituted, and when it is substituted, the substituent may be substituted at any accessible point of attachment, and the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, and carboxylate group.

The term "alkylene" refers to a saturated linear or branched aliphatic hydrocarbon group having 2 residues derived from the parent alkane by removal of two hydrogen atoms from the same carbon atom or two different carbon atoms; it is a linear or branched group containing 1 to 20 carbon atoms, preferably alkylene containing 1 to 12 carbon atoms, and more preferably alkylene containing 1 to 6 carbon atoms. Non-limiting examples of alkylene include, but are not limited to, methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), and the like. Alkylene may be substituted or unsubstituted, and when it is substituted, the substituent may be substituted at any accessible point of attachment.

The term "alkenylene" refers to a linear alkenyl group having 2 to 8 carbon atoms, preferably 2 to 6 carbon atoms, and more preferably 2 to 4 carbon atoms, and having at least one double bond at any position, including, for example, ethenylene, allylene, propenylene, butenylene, prenylene, butadienylene, pentenylene, pentadienylene, hexenylene, hexadienylene, and the like.

The term "alkynylene" refers to a linear alkynylene group having 2 to 8 carbon atoms, preferably 2 to 6 carbon atoms, and more preferably 2 to 4 carbon atoms, and having at least one triple bond at any position, including, for example, ethynylene, propynylene, butynelene, pentynylene, hexynylene, and the like.

The term "cycloalkyl" refers to a saturated or partially unsaturated monocyclic or polycyclic hydrocarbon substituent; the cycloalkyl ring contains 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spirocycloalkyl, fused cycloalkyl, and bridged cycloalkyl. "Carbocycle" refers to the ring system in cycloalkyl.

The term "spirocycloalkyl" refers to a 5- to 20-membered polycyclic group in which monocyclic rings share one carbon atom (referred to as a spiro atom); it may contain one or more double bonds, but none of the rings has a fully conjugated π-electron system. It is preferably 6- to 14-membered, and is more preferably 7- to 10-membered. According to the number of spiro atoms shared among rings, spirocycloalkyl may be monospirocycloalkyl, bispirocycloalkyl, or polyspirocycloalkyl, preferably monospirocycloalkyl and bispirocycloalkyl, and more preferably 4-membered/4-membered, 4-membered/5-membered, 4-membered/6-membered, 5-membered/5-membered, or 5-membered/6-membered monospirocycloalkyl. "Spirocarbocycle" refers to the ring system in spirocycloalkyl. Non-limiting examples of spirocycloalkyl include:

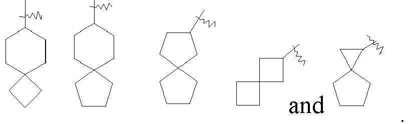

The term "fused cycloalkyl" refers to a 5- to 20-membered all-carbon polycyclic group in which each ring in the system shares a pair of adjacent carbon atoms with other rings in the system, wherein one or more rings may contain one or more double bonds, but none of them has a fully conjugated π-electron system. It is preferably 6- to 14-membered, and is more preferably 7- to 10-membered. According to the number of constituent rings, it may be bicyclic, tricyclic, tetracyclic, or polycyclic fused cycloalkyl, preferably bicyclic or tricyclic, and more preferably 5-membered/5-membered or 5-membered/6-membered bicycloalkyl. "Fused carbocycle" refers to the ring system in fused cycloalkyl. Non-limiting examples of fused cycloalkyl include:

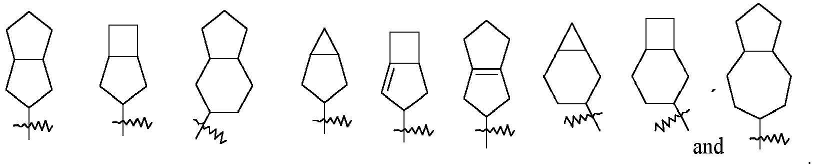

The term "bridged cycloalkyl" refers to a 5- to 20-membered all-carbon polycyclic group in which any two rings share two carbon atoms that are not directly connected; it may contain one or more double bonds, but none of the rings has a fully conjugated π-electron system. It is preferably 6- to 14-membered, and is more preferably 7- to 10-membered. According to the number of constituent rings, it may be bicyclic, tricyclic, tetracyclic, or polycyclic bridged cycloalkyl, preferably bicyclic, tricyclic, or tetracyclic, and more preferably bicyclic or tricyclic. Non-limiting examples of bridged cycloalkyl include:

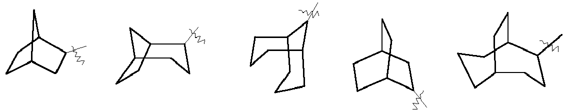

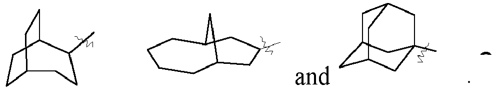

The cycloalkyl ring may be fused to an aryl, heteroaryl, or heterocycloalkyl ring, wherein the ring attached to the parent structure is cycloalkyl; non-limiting examples include indanyl, tetrahydronaphthyl, benzocycloheptyl, and the like. Cycloalkyl may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, and carboxylate group.

The term "heterocyclyl" refers to a saturated or partially unsaturated, monocyclic or polycyclic hydrocarbon substituent, which contains 3 to 20 ring atoms, one or more of which are heteroatoms selected from the group consisting of nitrogen, oxygen, and S(O)ₘ (where m is an integer from 0 to 2), but does not contain a ring moiety of -O-O-, -O-S-, or -S-S-, and the other ring atoms are carbon atoms. It preferably contains 3 to 12 ring atoms, 1 to 4 of which are heteroatoms; more preferably, it contains 3 to 6 ring atoms. Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, and the like, preferably piperidinyl and pyrrolidinyl. Polycyclic heterocyclyl includes spiroheterocyclyl, fused heterocyclyl, and bridged heterocyclyl. "Heterocycle" refers to the ring system in heterocyclyl.

The term "spiroheterocyclyl" refers to a 5- to 20-membered polycyclic heterocyclyl group in which monocyclic rings share one atom (referred to as a spiro atom), wherein one or more ring atoms are heteroatoms selected from the group consisting of nitrogen, oxygen, and S(O)ₘ (where m is an integer of 0 to 2), and the other ring atoms are carbon atoms. It may contain one or more double bonds, but none of the rings has a fully conjugated π-electron system. It is preferably 6- to 14-membered, and is more preferably 7- to 10-membered. According to the number of spiro atoms shared among rings, spiroheterocyclyl may be monospiroheterocyclyl, bispiroheterocyclyl, or polyspiroheterocyclyl, preferably, monospiroheterocyclyl and bispiroheterocyclyl, and more preferably 3-membered/4-membered, 3-membered/5-membered, 3-membered/6-membered, 4-membered/4-membered, 4-membered/5-membered, 4-membered/6-membered, 5-membered/5-membered, 5-membered/6-membered, or 6-membered/6-membered monospiroheterocyclyl. "Spiroheterocycle" refers to the ring system in spiroheterocyclyl. Non-limiting examples of spiroheterocyclyl include:

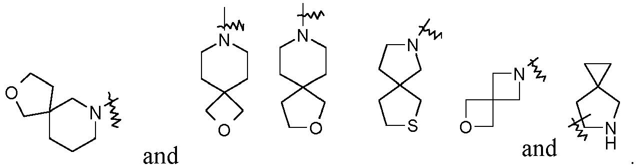

The term "fused heterocyclyl" refers to a 5- to 20-membered polycyclic heterocyclyl group in which each ring in the system shares a pair of adjacent atoms with the other rings in the system, wherein one or more rings may contain one or more double bonds, but none of the rings has a fully conjugated π-electron system; one or more ring atoms are heteroatoms selected from the group consisting of nitrogen, oxygen, and S(O)ₘ (where m is an integer of 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic fused heterocyclyl, preferably bicyclic or tricyclic, and more preferably 5-membered/5-membered or 5-membered/6-membered bicyclic fused heterocyclyl. "Fused heterocycle" refers to the ring system in fused heterocyclyl. Non-limiting examples of fused heterocyclyl include:

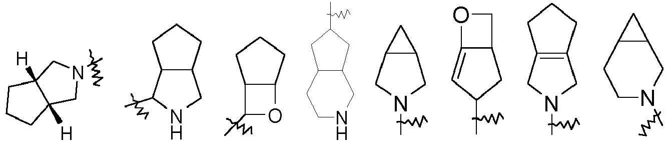

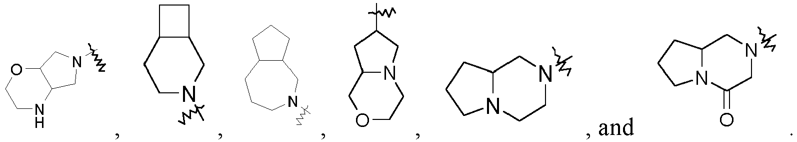

The term "bridged heterocyclyl" refers to a 5- to 14-membered polycyclic heterocyclyl group in which any two rings share two atoms that are not directly connected; it may contain one or more double bonds, but none of the rings has a fully conjugated π-electron system, wherein one or more ring atoms are heteroatoms selected from the group consisting of nitrogen, oxygen, and S(O)ₘ (where m is an integer of 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic bridged heterocyclyl, preferably bicyclic, tricyclic, or tetracyclic, and more preferably bicyclic or tricyclic. Non-limiting examples of bridged heterocyclyl include:

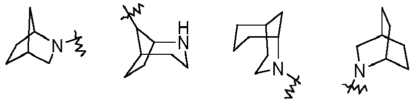

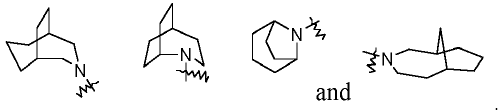

The heterocyclyl ring may be fused to an aryl, heteroaryl, or cycloalkyl ring, wherein the ring attached to the parent structure is heterocyclyl; its non-limiting examples include: 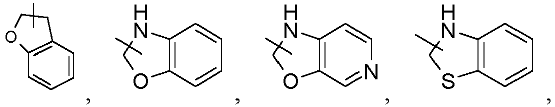 etc.

Heterocyclyl may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, and carboxylate group.

The term "aryl" refers to a 6- to 14-membered, preferably 6- to 10-membered, all-carbon monocyclic or fused polycyclic (i.e., rings that share a pair of adjacent carbon atoms) group having a conjugated π-electron system, such as phenyl and naphthyl. The aryl ring may be fused to a heteroaryl, heterocyclyl, or cycloalkyl ring, wherein the ring attached to the parent structure is the aryl ring. "Aromatic ring" refers to the ring system in aryl. Non-limiting examples of aryl include:

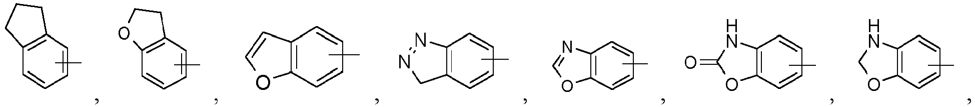

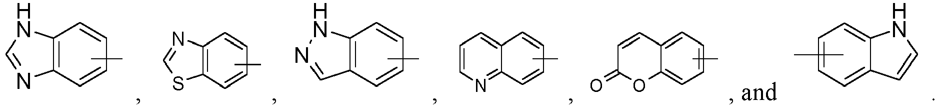

Aryl may be substituted or unsubstituted, and when it is substituted, the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, and carboxylate group, preferably phenyl.

The term "fused cycloaryl" may be an unsaturated aromatic fused ring structure containing 8-14 ring atoms, preferably 8-12 ring atoms, formed by connecting two or more ring structures that share two adjacent atoms with each other, including, for example, all unsaturated fused cycloaryl groups such as naphthalene and phenanthrene, and partially saturated fused cycloaryl groups such as benzo 3-8 membered saturated monocyclic cycloalkyl and benzo 3-8 membered partially saturated monocyclic cycloalkyl. "Fused aromatic ring" refers to the ring system in fused cycloaryl. Specific examples of fused cycloaryl include 2,3-dihydro-1H-indenyl, IH-indenyl, 1,2,3,4-tetrahydronaphthyl, 1,4-dihydronaphthyl, and the like.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Heteroaryl is preferably 5- to 12-membered, e.g., imidazolyl, furyl, thienyl, thiazolyl, pyrazolyl, oxazolyl, pyrrolyl, tetrazolyl, pyridyl, pyrimidinyl, thiadiazole, pyrazinyl, and the like, preferably imidazolyl, pyrazolyl, pyrimidinyl, or thiazolyl, and more preferably pyrazolyl or thiazolyl. The heteroaryl ring may be fused to an aryl, heterocyclyl, or cycloalkyl ring, wherein the ring attached to the parent structure is the heteroaryl ring. "Heteroaromatic ring" refers to the ring system in heteroaryl. Non-limiting examples of heteroaryl include:

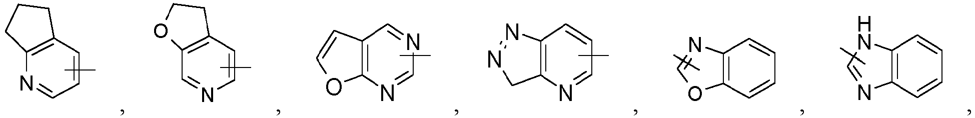

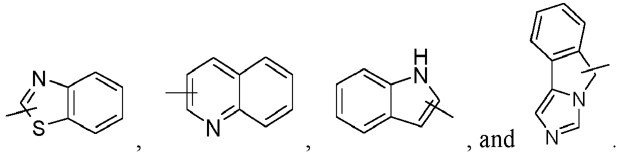

Heteroaryl may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, and carboxylate group.

The term "fused heteroaryl" may be an unsaturated aromatic fused ring structure containing 5-14 ring atoms (at least one heteroatom) formed by connecting two or more ring structures that share two adjacent atoms with each other, including the case where a carbon atom, a nitrogen atom, and a sulfur atom may be oxidized, preferably "5-12 membered fused heteroaryl", "7-12 membered fused heteroaryl", "9-12 membered fused heteroaryl", and the like, for example, benzofuranyl, benzoisothiafuranyl, benzothienyl, indolyl, isoindolyl, benzoxazolyl, benzimidazolyl, indazolyl, benzotriazolyl, quinolyl, 2-quinolinone, 4-quinolinone, 1-isoquinolinone, isoquinolinyl, acridinyl, phenanthridinyl, benzopyridazinyl, phthalazinyl, quinazolinyl, quinoxalinyl, phenazinyl, pteridinyl, purinyl, naphthyridinyl, phenazine, phenothiazine, and the like. "Fused heteroaromatic ring" refers to the ring system in fused heteroaryl.

Fused heteroaryl may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, and carboxylate group.

The cycloalkyl, heterocyclyl, aryl, and heteroaryl described above have 1 residue derived from the parent ring by removal of one hydrogen atom from a ring atom, or 2 residues derived from the parent ring by removal of two hydrogen atoms from the same ring atom or two different ring atoms, i.e., "divalent cycloalkyl", "divalent heterocyclyl", "arylene", or "heteroarylene".

The term "alkoxy" refers to -O-(alkyl) and -O-(unsubstituted cycloalkyl), wherein the alkyl is as defined above. Non-limiting examples of alkoxy include: methoxy, ethoxy, propoxy, butoxy, cyclopropyloxy, cyclobutoxy, cyclopentyloxy, and cyclohexyloxy. Alkoxy may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, and carboxylate group.

The term "alkylthio" refers to -S-(alkyl) and -S-(unsubstituted cycloalkyl), wherein the alkyl is as defined above. Non-limiting examples of alkylthio include: methylthio, ethylthio, propylthio, butylthio, cyclopropylthio, cyclobutylthio, cyclopentylthio, and cyclohexylthio. Alkylthio may be optionally substituted or unsubstituted, and when it is substituted, the substituent is preferably one or more of the following groups; it is substituted with one or more substituents independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "hydroxyalkyl" refers to an alkyl group substituted with hydroxy, wherein the alkyl group is as defined above.

The term "haloalkyl" refers to an alkyl group substituted with halogen, wherein the alkyl group is as defined above.

The term "deuterated alkyl" refers to an alkyl group substituted with a deuterium atom, wherein the alkyl group is as defined above.

The term "hydroxy" refers to the -OH group.

The term "oxo" refers to the =O group. For example, a carbon atom is connected to an oxygen atom via a double bond to form a ketone or aldehyde group.

The term "thio" refers to the =S group. For example, a carbon atom is connected to a sulfur atom via a double bond to form thiocarbonyl-C(S)-.

The term "halogen" refers to fluorine, chlorine, bromine, or iodine.

The term "amino" refers to -NH₂.

The term "cyano" refers to -CN.

The term "nitro" refers to -NO₂.

The term "carboxyl" refers to -C(O)OH.

The term "aldehyde" refers to -CHO.

The term "carboxylate group" refers to -C(O)O(alkyl) or -C(O)O(cycloalkyl), wherein the alkyl and cycloalkyl are as defined above.

The term "acyl halide" refers to a compound containing the group -C(O)-halogen.

The term "sulfonyl" refers to -S(O)(O)-.

The term "sulfinyl" refers to -S(O)-.

"Isosteres" of a chemical group are other chemical groups that exhibit the same or similar properties. For example, tetrazole is an isostere of carboxylic acid because it mimics the properties of carboxylic acid even though they have very different molecular formulas. Tetrazole is one of many possible isosteric replacements for carboxylic acid. Other carboxylic acid isosteres contemplated include -SO₃H, -SO₂HNR, -PO₂(R)₂, -PO₃(R)₂, -CONHNHSO₂R, -COHNSO₂R, and -CONRCN, where R is selected from the group consisting of hydrogen, alkyl, alkenyl, alkynyl, cycloalkyl, aryl, heteroaryl, and heterocyclyl as defined herein. In addition, carboxylic acid isosteres may include 5- to 7-membered carbocycles or heterocycles containing any combination of CH₂, O, S, or N in any chemically stable oxidation state, where any of the atoms of the ring structures are optionally substituted in one or more positions. It is also contemplated that when chemical substituents are added to a carboxylic isostere, the compound retains the properties of a carboxylic isostere. It is contemplated that when a carboxylic isostere is optionally substituted with one or more moieties selected from R as defined above, the substitution and substitution position are selected such that it does not eliminate the carboxylic acid isosteric properties of the compound. Similarly, it is also contemplated that if one or more R substituents would destroy the carboxylic acid isosteric properties of the compound, the placement of such substituents on a carbocyclic or heterocyclic carboxylic acid isostere is not a substitution at one or more atoms that maintain or are integral to the carboxylic acid isosteric properties of the compound.

"Optional" or "optionally" means that the event or circumstance subsequently described may, but does not necessarily, occur and that the description includes instances where the event or circumstance occurs or does not occur. For example, "a heterocyclyl group optionally substituted with alkyl" means that the alkyl may, but does not necessarily, exist and that the description includes instances where the heterocyclyl group is or is not substituted with the alkyl.

"Substituted" means that one or more, preferably up to 5, more preferably 1 to 3 hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort. For example, it may be unstable when an amino or hydroxy group having free hydrogen is bound to a carbon atom having an unsaturated (e.g., olefinic) bond.

In the chemical structure of the compound described herein, a " 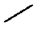 " bond is not specified with a configuration; that is, a " 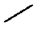 " bond may be " 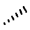 " or " 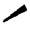 ", or includes both " 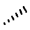 " and " 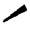 " configurations. In the chemical structure of the compound described herein, a bond " 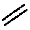 " is not specified with a configuration; that is, it may be in a Z configuration or an E configuration, or contains both configurations.

Although all of the above structural formulae are drawn as certain isomeric forms for the sake of simplicity, the present disclosure may include all isomers, such as tautomers, rotamers, geometric isomers, diastereomers, racemates, and enantiomers.

Tautomers are structural isomers of organic compounds that readily interconvert by a chemical reaction called tautomerization. This reaction often results in the formal migration of hydrogen atoms or protons accompanied by the conversion of a single bond to an adjacent double bond. Some common tautomeric pairs include: keto-enol and lactam-lactim. An example of a lactam-lactim equilibrium is present between A and B as shown below.

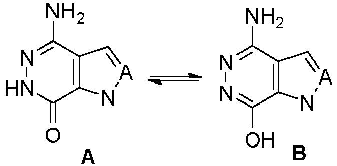

Additionally, after the chelating ring (DOTA-like ring) complexes with metal ions, two conformations, square antiprism (SAP) and twisted square antiprism (TSAP), are formed in the solution; therefore, they can form tautomers through the rotation of the chelating ring's pendent arms or the flipping of the ring; furthermore, the flipping of the side chain attached to the chelating ring can also result in the formation of tautomers. For specific explanations, see the journals (Dalton Trans. 2018, 47(31):10360; Dalton Trans. 2016, 45(11), 4673; Nature Communication 2018, 9:857; and Bioconjugate Chem. 2015, 26 (2), 338.).

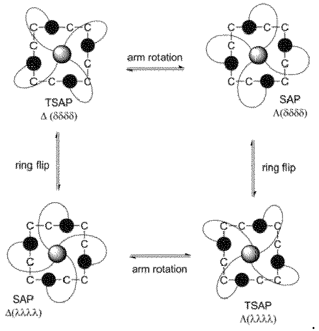

All compounds in the present disclosure can be drawn as form A or form B. All tautomeric forms are within the scope of the present disclosure. The names of the compounds do not exclude any tautomers.

Any isotopically-labeled derivative of the compound or the pharmaceutically acceptable salt or the isomer thereof of the present disclosure is encompassed by the present disclosure. Atoms that can be isotopically labeled include, but are not limited to, hydrogen, carbon, nitrogen, oxygen, phosphorus, fluorine, chlorine, iodine, and the like. They may be separately replaced by the isotopes ²H (D), ³H, ¹¹C, ¹³C, ¹⁴C, ¹⁵N, ¹⁸F, ³¹P, ³²P, ³⁵S, ³⁶Cl, ¹²⁵I, etc. Unless otherwise stated, when a position is specifically designated as deuterium (D), that position shall be understood to be deuterium having an abundance that is at least 3000 times greater than the natural abundance of deuterium (which is 0.015%) (i.e., at least 45% deuterium incorporation).

"Pharmaceutical composition" refers to a mixture containing one or more of the compounds described herein or a physiologically and pharmaceutically acceptable salt or pro-drug thereof, and other chemical components, for example physiologically and pharmaceutically acceptable carriers and excipients. The pharmaceutical composition is intended to promote the administration to an organism, so as to facilitate the absorption of the active ingredient, thereby exerting biological activity.

### BRIEF DESCRIPTION OF THE DRAWINGS

FIG. 1 shows a comparison of the enzyme activity experiments of PSMA-617, compound v, and compound x.
FIG. 2 shows 2-h biodistributions of 68Ga-v and 68Ga-x in LnCaP tumor-bearing mice.
FIG. 3 shows curves of the metabolisms of ⁶⁸Ga-PSMA-617 and ⁶⁸Ga-v (Example 9) in the blood of normal mice.

### DETAILED DESCRIPTION

The present disclosure is explained below in more detail with reference to examples. The examples of the present disclosure are only used to illustrate the technical solutions of the present disclosure, and the essence and scope of the present disclosure are not limited to these examples. Unless otherwise specified, all the starting materials used in the present disclosure are normal, commercially available products.

The NMR shifts (δ) are given in 10⁻⁶ (ppm). The NMR analyses were performed on a Bruker AVANCE-400 nuclear magnetic resonance instrument, with dimethyl sulfoxide-D6 (DMSO-d⁶), chloroform-D (CDCl₃), and methanol-D4 (CD₃OD) as solvents and tetramethylsilane (TMS) as an internal standard.

The MS analyses were performed on a Shimadzu 2010 Mass Spectrometer or Agilent 6110A MSD Mass Spectrometer.

The HPLC analyses were performed on Shimadzu LC-20A systems, Shimadzu LC-2010HT series, or Agilent 1200 LC high-performance liquid chromatograph (Ultimate XB-C18 3.0 × 150 mm chromatography column or Xtimate C18 2.1 × 30 mm chromatography column).

In the chiral HPLC analyses, Chiralpak IC-3 100 × 4.6 mm I.D., 3 µm, Chiralpak AD-3 150 × 4.6 mm I.D., 3 µm, Chiralpak AD-3 50 × 4.6 mm I.D., 3 µm, Chiralpak AS-3 150 × 4.6 mm I.D., 3 µm, Chiralpak AS-3 100 × 4.6 mm I.D., 3 µm, ChiralCel OD-3 150 × 4.6 mm I.D., 3 µm, Chiralcel OD-3 100 × 4.6 mm I.D., 3 µm, ChiralCel OJ-H 150 × 4.6 mm I.D., 5 µm, and Chiralcel OJ-3 150 × 4.6 mm I.D., 3 µm chromatography columns were used. Yantai Huanghai HSGF254 or Qingdao GF254 silica gel plates, 0.15 mm-0.2 mm layer thickness, were used in the thin-layer chromatography (TLC) analyses and 0.4 mm-0.5 mm layer thickness in the TLC separations and purifications.

Yantai Huanghai silica gel of 100-200 mesh, 200-300 mesh, or 300-400 mesh was generally used as a carrier in the column chromatography purifications.

In the preparative chiral chromatography purifications, a DAICEL CHIRALPAK IC (250 mm × 30 mm, 10 µm) or Phenomenex-Amylose-1 (250 mm × 30 mm, 5 µm) column was used.

The CombiFlash preparative flash chromatograph used was Combiflash Rf150 (TELEDYNE ISCO).

The kinase mean inhibition rates and IC₅₀ values were measured using a NovoStar microplate reader (BMG, Germany).

Known starting materials in the present disclosure may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen gas.

The hydrogen atmosphere means that the reaction flask is connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization/hydrogen filling.

The microwave reactions were performed using a CEM Discover-S 908860 microwave reactor.

In the examples, the solutions refer to aqueous solutions unless otherwise specified.

In the examples, the reaction temperature is room temperature, i.e., 20 °C-30 °C, unless otherwise specified.

The monitoring of the reaction processes in the examples was conducted using thin-layer chromatography (TLC). The developing solvents for the reactions, the eluent systems of column chromatography for compound purification, and the developing solvent systems of thin-layer chromatography include: A: a dichloromethane/methanol system, B: a n-hexane/ethyl acetate system, C: a petroleum ether/ethyl acetate system, and D: a petroleum ether/ethyl acetate/methanol system. The volume ratio of the solvents was adjusted depending on the polarity of the compound, or adjusted by adding a small amount of basic or acidic reagents such as triethylamine and acetic acid.

The abbreviations used in the experiments below have the following meanings:
EtOAc (EA): ethyl acetate; DCM: dichloromethane; THF: tetrahydrofuran; DIPEA: *N,N-*diisopropylethylamine; PPTS: pyridinium p-toluenesulfonate; Boc: t-butoxycarbonyl; MeOH: methanol; HATU: 2-(7-azabenzotriazole)-N,N,N',N'-tetramethyluronium hexafluorophosphate; DIEA: N,N-diisopropylethylamine.

### Example 1

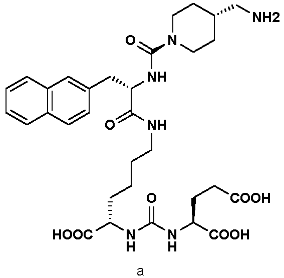

### (((S)-5-((S)-2-(4-(aminomethyl)piperidine-1-carboxamido)-3-(2-naphthyl)propanamido)-1-carboxy pentyl)carbamoyl)-L-glutamic acid

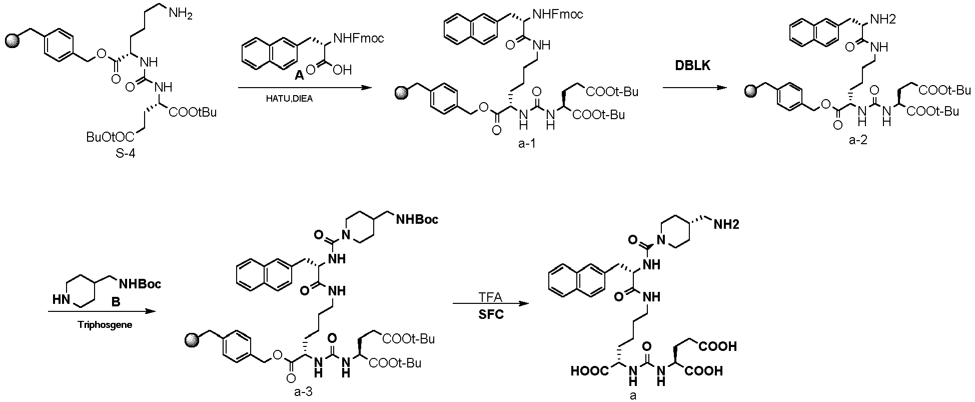

### Step 1 (preparation of resin compound S-4):

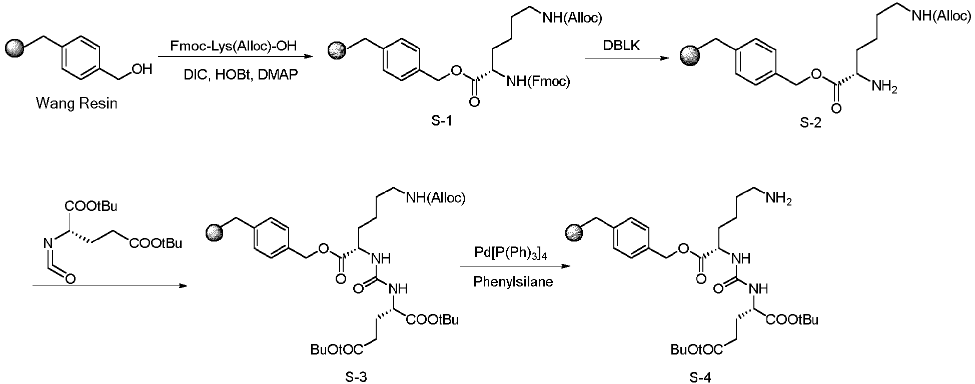

The starting materials and resin needed were taken out, placed in a desiccator, and equilibrated to room temperature. 25 g of Wang Resin (sub = 0.38 mmol/g, 8.75 mmol) (Wang Resin, purchased from Xi'an Sunresin Tech Ltd.) was weighed out and placed into a 500 mL single-necked flask, and 250 mL of DMF was added. The flask was placed in a shaker and shaken for 30 min. Fmoc-Lys(Alloc)-OH (19.8 g, 43.75 mmol) (N-[(9H-fluoren-9-methoxy)carbonyl]-N'-[(2-propenyloxy)carbonyl]-L-lysine, purchased from GL Biochem), DIC (5.5 g, 43.75 mmol), HOBt (13.0 g, 43.75 mmol) (1-hydroxybenzotriazole, purchased from Meryer), and DMAP (0.11 g, 0.875 mmol) (4-dimethylaminopyridine, purchased from Energy) were added, and the mixture was left to react on the shaker at room temperature for 23 h. The resin was transferred into a solid-phase reaction column, and the reaction solution was removed. The resin was washed 3 times with DMF, 300 mL per wash. The resin was blocked with 150 mL of pyridine:acetic anhydride = 1:1 (V:V). The resin was blocked for 8 h, and the peptide resin was shrunk and dried with methanol to give the product S-1 (8.75 mmol).

The product S-1 (7 mmol) was swollen with 150 mL of DMF at room temperature, and then 200 mL of 20% DBLK (a 20% piperidine/DMF solution, purchased from Energy) was added for deprotection (10 min). After the liquid was removed, another 200 mL of 20% DBLK was added for deprotection (10 min). A Kaiser test of the resin yielded blue. The resin was drained and washed with DMF until it was neutral, and the product S-2 was obtained.

The product S-2 (7 mmol) was added to a glutamyl isocyanate reaction solution at room temperature. The reaction was stirred slowly on a thermostatic shaker for 18 h, and a Kaiser test of the resin yielded no color change. The resin was transferred into a solid-phase reaction column, and the reaction solution was removed. The resin was washed 3 times with DMF, 300 mL per wash, to give the product S-3.

The product S-3 (7 mmol) was added to a reaction column at room temperature. Phenylsilane (4.6 g, 42 mmol) and tetrakis(triphenylphosphine)palladium(0) (0.81 g, 0.7 mmol) were dissolved in 180 mL of DCM, and the resulting solution was added to the reaction column. Nitrogen was bubbled through the mixture for 0.5 h, and the solvent was removed. The procedure was repeated 2 times, and a Kaiser test of the resin yielded bluish black. After the reaction was complete, the solvent was removed. The resin was washed with DMF three times, drained, shrunk three times with methanol, and then dried *in vacuo* at 30 °C for 2 h to give the product S-4 (22.2 g, 7 mmol) for later use.

### Step 2:

The resin compound S-4 (4.0 g, 1.28 mmol) was swollen with dichloromethane (purchased from Sinopharm Chemical Reagent Co., Ltd.) at room temperature for 0.5 h, drained, washed three times with DMF, and drained for later use.

Fmoc-2-NAL-OH (1.68 g, 3.84 mmol) (Fmoc-3-(2-naphthyl)-L-alanine, purchased from Meryer), HOBt (0.52 g, 3.84 mmol) (1-hydroxybenzotriazole, purchased from Meryer), HATU (1.46 g, 3.84 mmol) 2-(7-azabenzotriazole)-N,N,N',N'-tetramethyluronium hexafluorophosphate, purchased from Macklin), and DIEA (1.01 g, 7.68 mmol) (N,N-diisopropylethylamine, purchased from Energy) were weighed out and dissolved in DMF (25 mL) (N,N-dimethylformamide, purchased from Energy), and the resulting solution was then added to a solid-phase reaction column containing compound S-4. After 2 h of reaction, a Kaiser test of the resin yielded no color change. The reaction solution was removed, and the resin was washed three times with DMF to give the title product a-1.

### Step 3:

At room temperature, 40 mL of 20% DBLK (a 20% piperidine/DMF solution, purchased from Energy) was added to a solid-phase reaction column containing a-1 for deprotection (10 min). After the liquid was removed, another 40 mL of 20% DBLK was added for deprotection (10 min). A Kaiser test of the resin yielded blue. The resin was drained and washed with DMF until it was neutral, and the product, compound a-2, was obtained.

### Step 4:

Compound a-2 (1.0 mmol) and triphosgene (0.2 g, 0.68 mmoL) were added to 6 mL of DCM solution at room temperature. The mixture was cooled to 0 °C, and DIEA (0.65 g, 5 mmol) was added dropwise. After 2 h of reaction at that temperature, a Kaiser test of the resin yielded no color change. 4-Boc-aminomethylpiperidine was added, and the mixture was warmed to room temperature and left to react for 3 h. The reaction solution was removed, and the resin was washed three times with DMF (N,N-dimethylformamide), shrunk three times with methanol, and then dried *in vacuo* at 30 °C for 2 h to give the product, compound a-3, for later use.

### Step 5:

A 40 mL cleavage solution (TFA (trifluoroacetic acid):H₂O:Tis (triisopropylsilane, purchased from Macklin) = 95:2.5:2.5) was prepared at room temperature, and the peptide resin compound 4 was added with stirring. The mixture was left to react for 2 h. Then the mixture was filtered under reduced pressure. The resin was removed, and the filtrate was concentrated by rotary evaporation. The concentrate was added to 100 mL of isopropyl ether. The mixture was filtered under reduced pressure, and the filter cake was dried under reduced pressure to give a crude peptide (0.40 g, 60.9% yield). The crude product was purified by high-pressure preparative liquid chromatography to give the title product, compound a (105 mg, 26.2% yield).
MS m/z (ESI): 657.3 [M+1]⁺
1H NMR (400 MHz, Deuterium Oxide) δ 7.82 (t, J = 9.1 Hz, 3H), 7.64 (s, 1H), 7.46 (s, 2H), 7.37 (d, J = 8.4 Hz, 1H), 4.51 (t, J = 8.1 Hz, 1H), 4.14 (s, 1H), 3.85 (dd, J = 30.1, 11.1 Hz, 2H), 3.71 (d, J = 13.8 Hz, 1H), 3.22 (dd, J = 13.6, 7.5 Hz, 1H), 3.14-3.01 (m, 1H), 2.97-2.83 (m, 1H), 2.67 (dt, J = 33.6, 12.7 Hz, 2H), 2.40 (dt, J = 15.4, 6.9 Hz,4H), 2.12-1.97 (m, 1H), 1.84 (dd, J = 14.3, 7.5 Hz, 1H), 1.68 (s, 1H), 1.49 (d, J = 19.0 Hz, 4H), 1.36 (s, 1H), 1.13 (d, J = 7.4 Hz, 2H), 0.93 (s, 2H), 0.66 (d, J = 12.2 Hz, 1H), 0.54 (s, 1H).

### Example 2

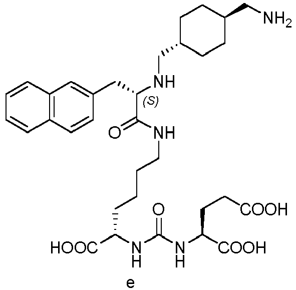

### (((S)-5-((S)-2-((((1R,4S)-4-(aminomethyl)cyclohexyl)methyl)amino)-3-(2-naphthyl)propanami do)-1-carboxypentyl)carbamoyl)-L-glutamic acid

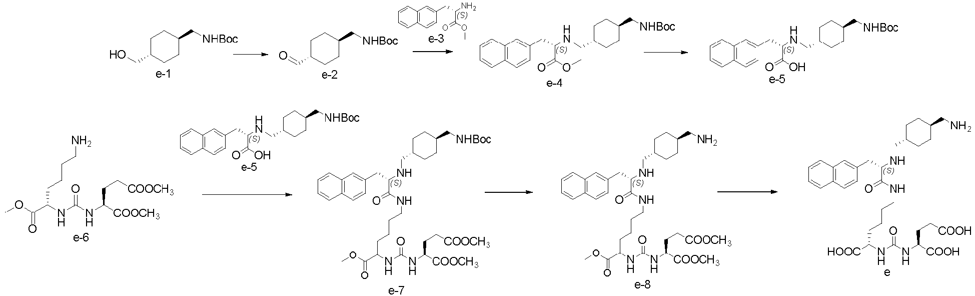

### Step 1:

Trans-4-(Boc-aminomethyl)cyclohexanemethanol compound e-1 (3 g, 12 mmol) was dissolved in 60 mL of DCM at room temperature. The solution was cooled to -60 °C, and a DMP/DCM solution (7.95 g, 18 mmol, DCM 60 mL) was added. After the addition, the mixture was naturally warmed to room temperature and stirred for 4 h. TLC monitoring showed the reaction was complete. The reaction solution was washed with 100 mL of an aqueous solution of Na₂CO₃ and Na₂S₂O₃ and then with 100 mL of saturated sodium chloride solution, dried over anhydrous sodium sulfate, and filtered. The filtrate was concentrated under reduced pressure and purified by silica gel column chromatography eluting with an eluent system of n-heptane/ethyl ester = 10:1-4:1 to give compound e-2 (1.8 g, 59.7% yield).
MS m/z (ESI): 242.3 [M+1]⁺

### Step 2:

Compound e-3 (1.56 g, 6.8 mmol) and compound e-2 (1.48 g, 6.1 mmol) were dissolved in 40 mL of a solution of DCM/THF (V 1: V2 = 1:1) at room temperature, and the resulting solution was stirred for 2 h. Sodium cyanoborohydride (0.5 g, 7.9 mmol) and acetic acid (0.3 mL) were slowly added, and the mixture was stirred for 3 h. The reaction solution was washed with water and extracted with DCM (40 mL × 3). The organic phases were combined, washed with 50 mL of saturated sodium chloride solution, dried over anhydrous sodium sulfate, and filtered. The filtrate was concentrated under reduced pressure and purified by silica gel column chromatography eluting with an eluent system of n-heptane/ethyl ester = 10:1-4:1 to give compound e-4 (1.54 g, 55.6% yield).
MS m/z (ESI): 455.3 [M+1]⁺

### Step 3:

Compound e-4 (1.5 g, 3.3 mmol) was dissolved in 12 mL of THF and 5 mL of water at room temperature, and lithium hydroxide (0.24 g, 9.9 mmol) was added. After the addition, the mixture was left to react at room temperature overnight. 10 mL of water was added to the reaction solution, and the mixture was extracted with ethyl acetate (10 mL × 2). The aqueous phases were combined and cooled to 0 °C in an ice bath, and the pH was adjusted to 3-4 with 0.5 N citric acid. A solid precipitated. 100 mL of H₂O and 50 mL of DCM were added, and the mixture was stirred for 0.5 h. A pH test showed the pH did not change. The mixture was filtered, and the filter cake was dried *in vacuo* (40 °C, 4 h) to constant weight to give the product, compound e-5 (1.1 g, 75.9% yield).
MS m/z (ESI): 441.3 [M+1]⁺

### Step 4:

Compound e-5 (0.13 g, 0.3 mmol), HATU (0.16 g, 0.42 mmol), DIEA (0.18 g, 1.41 mmol), and DCM (1 mL) were added to a reaction flask at room temperature and stirred to form a clear solution. Compound e-6 was added, and the mixture was stirred overnight. The reaction solution was washed with water and extracted with EA (30 mL × 3). The organic phases were combined, washed with 50 mL of saturated sodium chloride solution, dried over anhydrous sodium sulfate, and filtered. The filtrate was concentrated under reduced pressure and purified by silica gel column chromatography eluting with an eluent system of n-heptane/ethyl ester = EA (0%-90%) to give the product, compound e-7 (0.13 g, 59.4% yield).
MS m/z (ESI): 784.4 [M+1]⁺

### Step 5:

Compound e-7 (0.13 g, 0.16 mmol) was dissolved in 2 mL of ethyl acetate at room temperature, and a 2 M HCL/EA solution (2 mL, 4 mmol) was added with stirring. The mixture was stirred for 2 h. TLC monitoring showed the reaction was complete. The reaction solution was concentrated under reduced pressure to constant weight to give the product, compound e-8 (0.11 g, 92% yield).
MS m/z (ESI): 684.3 [M+1]⁺

### Step 6:

Compound e-8 (0.11 g, 0.16 mmol) was dissolved in 2 mL of THF and 1 mL of water at room temperature, and lithium hydroxide (29 mg, 1.2 mmol) was added. After the addition, the mixture was left to react at room temperature overnight. TFA was added to the reaction solution to adjust the pH to 2-3. The solution was stirred for 0.5 h, and a pH test showed the pH did not change. The reaction solution was purified by high-pressure preparative liquid chromatography to give the title product, compound e (80 mg, 78.4% yield).
MS m/z (ESI): 640.3 [M-1]⁻
1H NMR (400 MHz, Deuterium Oxide) δ 7.94 (t, J = 6.7 Hz, 3H), 7.91-7.80 (m, 1H), 7.54 (p, J = 7.2 Hz, 1H), 7.46 (t, J = 7.8 Hz, 1H), 7.38 (d, J = 7.1 Hz, 1H), 4.18 (dt, J = 9.1, 5.6 Hz, 2H), 4.05 (d, J = 7.6 Hz, 2H), 3.69 (dd, J = 13.3, 4.9 Hz, 2H), 3.53 (t, J = 12.3 Hz, 2H), 2.94-2.70 (m, 3H), 2.42 (s, 1H), 2.08 (s, 2H), 1.80 (s, 1H), 1.68 (s, 2H), 1.58 (s, 2H), 1.39 (d, J = 9.7 Hz, 2H), 1.27 (s, 3H), 1.02 (q, J = 10.5, 9.9 Hz, 3H), 0.63 (s, 2H), 0.46 (s, 2H).

### Example 3

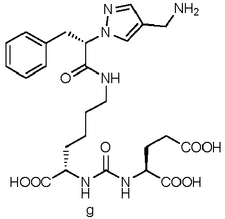

### (((S)-5-((S)-2-(4-(aminomethyl)-1-pyrazolyl)-3-phenylpropanamido)-1-carboxypentyl)carbam oyl)-L-glutamic acid

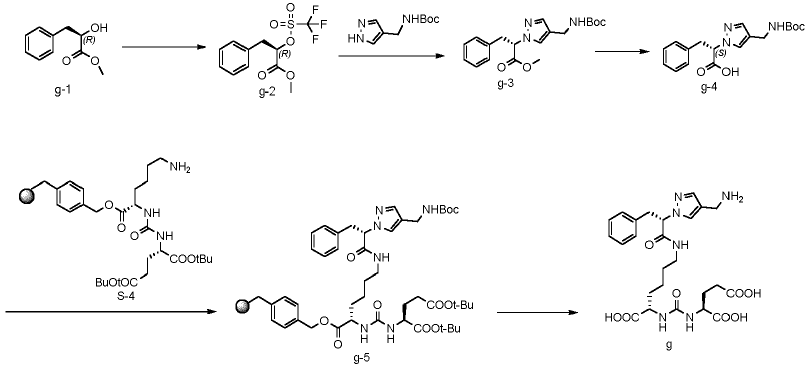

### Step 1:

Boc-L-tyrosine methyl ester compound g-1 (0.5 g, 2.78 mmol) was dissolved in 120 mL of DCM at room temperature, and pyridine (1.2 mL, 14.61 mmol) was added. The mixture was cooled to 0 °C, and trifluoromethanesulfonic anhydride (2.4 mL, 14.10 mmol) was added. After the addition, the mixture was naturally warmed to room temperature and stirred for 3 h. TLC monitoring showed the reaction was complete. The reaction solution was washed with 100 mL of saturated sodium bicarbonate solution, with 100 mL of 1 N HCL, and then with 100 mL of saturated sodium chloride solution, dried over anhydrous sodium sulfate, and filtered. The filtrate was concentrated to dryness under reduced pressure to give compound g-2 (0.84 g, 99.0% yield).

### Step 2:

4-(Boc-aminomethyl)pyrazole (0.61 g, 3.01 mmol) was dissolved in 25 mL of DCM at room temperature, and DIEA (0.49 g, 3.72 mmol) was added. The mixture was stirred for 1 h. A solution of compound 2 (0.84 g, 2.69 mmol) in DCM was added dropwise, and the mixture was stirred overnight. The reaction solution was washed with 30 mL of saturated sodium bicarbonate solution and then with 30 mL of saturated sodium chloride solution, dried over anhydrous sodium sulfate, and filtered. The filtrate was concentrated under reduced pressure and purified by silica gel column chromatography eluting with an eluent system of n-heptane/ethyl ester = 10:1-3:1 to give compound g-3 (0.51 g, 48.4% yield).
MS m/z (ESI): 360.2 [M+1]⁺

### Step 3:

Compound g-3 (0.51 g, 1.4 mmol) was dissolved in 1.5 mL of THF and 1 mL of water at room temperature, and lithium hydroxide was added. After the addition, the mixture was left to react at room temperature overnight. 10 mL of water was added to the reaction solution, and the mixture was extracted with ethyl acetate (10 mL × 2). The aqueous phases were combined and cooled to 0 °C in an ice bath, and the pH was adjusted to 3-4 with 0.5 N citric acid. An ethyl ester (10 mL × 3) was added for extraction. The organic phases were combined, washed with saturated sodium chloride, dried over anhydrous sodium sulfate, and filtered. The filtrate was concentrated under reduced pressure to give the title product, compound g-4 (0.39 g, 80.0% yield).
MS m/z (ESI): 344.5 [M-1]⁻

### Step 4:

The resin compound S-4 prepared in Example 1 (1.1 g, 0.38 mmol) was swollen with DCM at room temperature for 0.5 h, drained, and washed three times with DMF for later use.

Compound g-4 (0.36 g, 1.04 mmol), HATU (0.4 g, 1.04 mmol), HOBt (0.14 g, 1.04 mmol), DIEA (0.27 g, 2.08 mmol), and DMF (10 mL) were added to a reaction flask, and the flask was shaken to form a clear solution. The swollen resin prepared in advance was added to the reaction flask, and the flask was shaken overnight. A Kaiser test of the resin yielded no color change. The reaction solution was removed, and the resin was washed three times with DMF, shrunk with methanol, and then dried for later use. The product, compound g-5, was obtained.

### Step 5:

A 10 mL cleavage solution (TFA:H2O:Tis = 95:2.5:2.5) was prepared at room temperature, and the peptide resin compound g-5 was added with stirring. The mixture was left to react for 2 h. Then the mixture was filtered under reduced pressure. The resin was removed, and the filtrate was concentrated by rotary evaporation. The concentrate was added to 40 mL of isopropyl ether, and a solid precipitated. The mixture was filtered under reduced pressure, and the resulting solid was dried under reduced pressure to give a crude peptide (0.15 g, 72.1% yield). The crude product was purified by high-pressure preparative liquid chromatography to give the title product, compound g (26 mg, 17.3% yield).
MS m/z (ESI): 547.8 [M+1]⁺
1H NMR (400 MHz, Deuterium Oxide) δ 7.77 (s, 1H), 7.54 (s, 1H), 7.26-7.13 (m, 3H), 7.13-7.06 (m, 2H), 5.07 (t, J = 8.2 Hz, 1H), 4.17-4.08 (m, 1H), 3.95 (s, 3H), 3.32 (d, J = 8.2 Hz, 2H), 3.06 (dt, J = 12.8, 6.2 Hz, 1H), 2.92 (dt, J = 13.4, 6.5 Hz, 1H), 2.37 (t, J = 7.3 Hz, 2H), 2.04 (dq, J = 13.1, 7.2 Hz, 1H), 1.83 (dq, J = 14.9, 7.3 Hz, 1H), 1.59 (s, 1H), 1.47 (dd, J = 9.4, 4.7 Hz, 1H), 1.25-1.17 (m, 2H), 0.98 (d, J = 6.7 Hz, 2H).

### Example 4

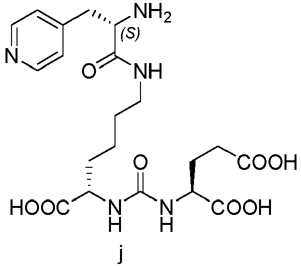

### (((S)-5-((S)-2-amino-3-(4-pyridinyl)propanamido)-1-carboxypentyl)carbamoyl)-L-glutamic acid

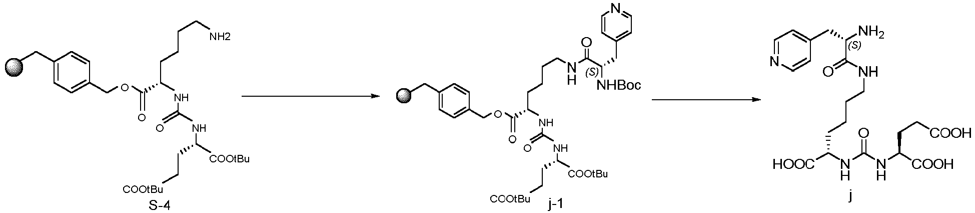

### Step 1:

The resin compound S-4 (1.7 g, 0.65 mmol) was swollen with DCM at room temperature for 0.5 h, drained, and washed three times with DMF for later use.

Boc-3-(4-pyridinyl)-L-alanine (0.54 g, 2.01 mmol), HATU (0.76 g, 2.01 mmol), HOBt (0.27 g, 2.01 mmol), DIEA (0.52 g, 4.02 mmol), and DMF (15 mL) were added to a reaction flask, and the flask was shaken to form a clear solution. The swollen resin prepared in advance was added to the reaction flask, and the flask was shaken for 2 h. A Kaiser test of the resin yielded no color change. The reaction solution was removed, and the resin was washed three times with DMF, shrunk with methanol, and then dried for later use. The title product, compound j-1, was obtained.

### Step 2:

A 20 mL cleavage solution (TFA:H₂O:Tis = 95:2.5:2.5) was prepared at room temperature, and the peptide resin compound 2 was added with stirring. The mixture was left to react for 2 h. Then the mixture was filtered under reduced pressure. The resin was removed, and the filtrate was concentrated by rotary evaporation. The concentrate was added to 50 mL of isopropyl ether, and a solid precipitated. The mixture was filtered under reduced pressure, and the resulting solid was dried under reduced pressure to give a crude peptide (0.20 g, 66.7% yield). The crude product was purified by high-pressure preparative liquid chromatography to give the title product, compound j (75 mg, 37.5% yield).
MS m/z (ESI): 468.8 [M+1]⁺
1H NMR (400 MHz, Deuterium Oxide) δ 8.73-8.66 (m, 2H), 7.90 (d, J = 6.3 Hz, 2H), 4.17 (ddd, J = 23.5, 9.1, 5.6 Hz, 2H), 4.00 (dd, J = 8.9, 5.0 Hz, 1H), 3.50-3.30 (m, 2H), 3.08 (dt, J = 13.6, 6.8 Hz, 1H), 2.95 (dt, J = 13.5, 6.8 Hz, 1H), 2.40 (t, J = 7.3 Hz, 2H), 2.07 (dq, J = 13.2, 7.2 Hz, 1H), 1.85 (ddd, J = 16.1, 14.1, 7.1 Hz, 1H), 1.59 (dtd, J = 55.3, 14.5, 14.1, 7.9 Hz, 2H), 1.25 (s, 2H), 1.08 (d, J = 9.0 Hz, 2H).

### Example 5

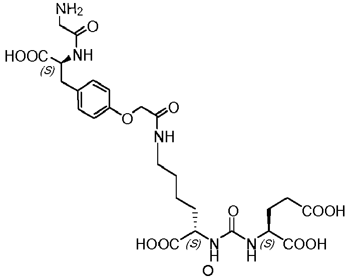

### (((S)-5-(2-(4-((S)-2-(2-aminoacetamido)-2-carboxyethyl)phenoxy)acetamido)-1-carboxypentyl) carbamoyl)-L-glutamic acid

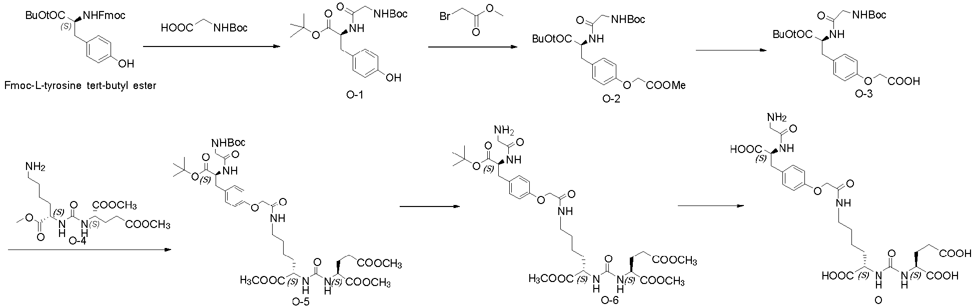

### Step 1:

Fmoc-L-tyrosine tert-butyl ester (5.0 g, 10.9 mmol) was dissolved in 50 mL of DMF at room temperature, and potassium carbonate (1.81 g, 13.1 mmol) was added. The mixture was stirred at room temperature for 12 h. Boc-glycine (2.29 g, 13.1 mmol) and HATU (4.98 g, 13.1 mmol) were added, and the mixture was cooled to 0 °C in an ice bath. DIEA was added dropwise, and the mixture was stirred at room temperature for 2 h. 100 mL of water was added to the reaction solution, and the mixture was extracted with ethyl acetate (100 mL × 2). The organic phases were combined, washed with saturated sodium bicarbonate solution (100 mL), with saturated ammonium chloride solution (100 mL × 2), and with saturated sodium chloride solution (100 mL × 2), dried over anhydrous sodium sulfate, and filtered. The filtrate was concentrated under reduced pressure and purified by silica gel column chromatography eluting with an eluent system of PE/EA = 100:1-50:50 to give the product, compound O-1 (3.8 g, 70% yield).
MS m/z (ESI): 395.4 [M+1]⁺

### Step 2:

Compound O-1 (3.8 g, 9.7 mmol) was dissolved in 40 mL of DMF at room temperature, and potassium carbonate (1.81 g, 13.1 mmol) and methyl bromoacetate (2.22 g, 14.5 mmol) were added. The mixture was heated to 80 °C and stirred for 5-10 h. 100 mL of water was added to the reaction solution, and the mixture was extracted with ethyl acetate (100 mL × 2). The organic phases were combined, washed with saturated sodium chloride solution (100 mL × 2), dried over anhydrous sodium sulfate, and filtered. The filtrate was concentrated under reduced pressure to give the product, compound O-2 (4.2 g, 110% yield).
MS m/z (ESI): 467.4 [M+1]⁺

### Step 3:

Compound O-2 (4.2 g, 9.0 mmol) was dissolved in 50 mL of THF and 50 mL of water at room temperature. The solution was cooled to 0 °C in an ice bath, and an aqueous solution of lithium hydroxide was slowly added. After the addition, the mixture was left to react at room temperature for 2 h. The reaction solution was extracted with ethyl acetate (100 mL × 2). The aqueous phases were combined and cooled to 0 °C in an ice bath, and the pH was adjusted to 3-4 with 0.5 N dilute hydrochloric acid. Ethyl acetate (100 mL × 2) was added for extraction. The organic phases were combined, dried over anhydrous sodium sulfate, and filtered. The filtrate was concentrated under reduced pressure and purified by silica gel column chromatography eluting with an eluent system of DCM/MeOH = 100:1-20:1 to give the product, compound O-3 (3.5 g, 80% yield).
MS m/z (ESI): 451.4 [M-1]⁻

### Step 4:

Compound O-3 (500 mg, 1.1 mmol) was dissolved in 30 mL of DCM at room temperature, and HATU was added. The mixture was cooled to 0 °C in an ice bath, and DIEA was added dropwise. The mixture was stirred at room temperature for 0.5 h. The mixture was cooled to 0 °C in an ice bath, and compound O-4 was added. The mixture was stirred at room temperature for 2-5 h. 100 mL of water was added to the reaction solution, and the mixture was extracted with DCM (100 mL × 2). The organic phases were combined, washed with saturated sodium bicarbonate solution (100 mL) and with saturated sodium chloride solution (100 mL × 2), dried over anhydrous sodium sulfate, and filtered. The filtrate was concentrated under reduced pressure and purified by silica gel column chromatography eluting with an eluent system of DCM/MeOH = 100:1-20:1 to give the product, compound O-5 (970 mg, 80% yield).
MS m/z (ESI): 796.5 [M+1]⁺

### Step 5:

Compound O-5 (970 mg, 1.22 mmol) was dissolved in 5 mL of ethyl acetate at room temperature, and a 2 N solution of hydrogen chloride in ethyl acetate (15 mL) was added. The mixture was stirred for 1-2 h. The reaction solution was concentrated under reduced pressure to give the product, compound O-6 (1.2 g, 110% yield).
MS m/z (ESI): 696.5 [M+1]⁺

### Step 6:

Compound O-6 (200 mg, 0.29 mmol) was dissolved in 10 mL of THF and 10 mL of water at room temperature. The solution was cooled to 0 °C in an ice bath, and lithium hydroxide (41.76 mg, 1.74 mmol) was slowly added. After the addition, the mixture was left to react at room temperature for 12 h. The reaction solution was extracted with ethyl acetate (20 mL × 2). The aqueous phases were combined and cooled to 0 °C in an ice bath, and the pH was adjusted to 2-3 with 1 N dilute hydrochloric acid. The mixture was purified by high-pressure preparative liquid chromatography to give the title product, compound O (20 mg, 20% yield).
MS m/z (ESI): 598.3 [M+1]⁺
1H NMR (400 MHz, D2O) δ 7.15 (d, J = 8.6 Hz, 2H), 6.85 (d, J = 8.7 Hz, 2H), 4.52 (s, 2H), 4.44 (dd, J = 8.9, 5.1 Hz, 1H), 4.09 (dd, J = 8.6, 5.0 Hz, 1H), 3.99 (dd, J = 8.2, 4.9 Hz, 1H), 3.73-3.58 (m, 3H), 3.18 (t, J = 6.6 Hz, 2H), 3.10-3.04 (m, 1H), 2.84 (dd, J = 14.6, 9.1 Hz, 1H), 2.37 (t, J = 7.3 Hz, 2H), 2.07-2.00 (m, 1H), 1.88-1.79 (m, 1H), 1.67 (s, 1H), 1.55 (d, J = 7.5 Hz, 1H), 1.46-1.39 (m, 2H), 1.20 (d, J = 15.5 Hz, 3H).

### Example 6

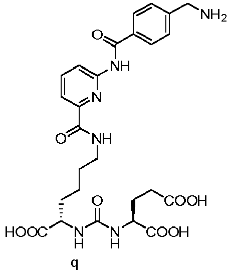

### (((S)-5-(6-(4-(aminomethyl)benzamido)picolinamido)-1-carboxypentyl)carbamoyl)-L-glutamic acid

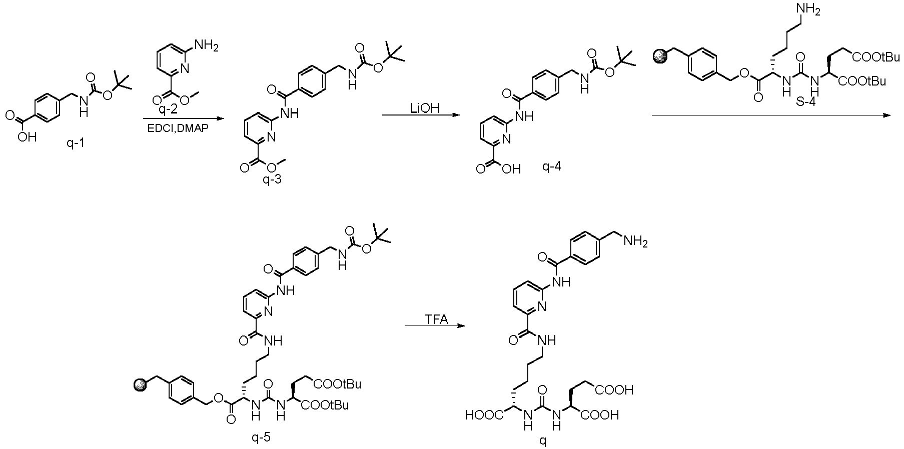

### Step 1:

Compound q-1 (3 g, 11.94 mmol) was dissolved in 60 mL of dichloromethane at room temperature, and EDCI (5.49 g, 28.66 mmol) (1-(3-dimethylaminopropyl)-3-ethylcarbodiimide hydrochloride, purchased from Macklin) and DMAP (4.37 g, 35.82 mmol) were added. The mixture was stirred in a N₂ atmosphere for 20 min, and compound q-2 (2.17 g, 14.33 mmol) was added. The mixture was stirred at room temperature for 16-18 h. 60 mL of water was added to the reaction solution, and the mixture was extracted with dichloromethane (100 mL × 3). The organic phases were combined, with saturated sodium chloride solution (50 mL), dried over anhydrous sodium sulfate, and filtered. The filtrate was concentrated under reduced pressure, and the resulting residue was purified by silica gel column chromatography with an eluent system (PE/EA= 100%-50%) to give the product, compound q-3 (2.0 g, 43.5% yield).
MS m/z (ESI): 386.2 [M+1]⁺

### Step 2:

Compound q-3 (1 g, 2.59 mmol) was dissolved in 6 mL of tetrahydrofuran at room temperature, and 4 mL of an aqueous solution of LiOH (187 mg, 7.77 mmol) was added. The mixture was stirred at room temperature for 16-18 h. 20 mL of water was added to the reaction solution, and the mixture was extracted with ethyl acetate (50 mL × 2). The pH of the aqueous phase was adjusted to 3-4 with 0.5 mol/L citric acid, and the aqueous phase was then extracted with ethyl acetate (50 mL × 4). The organic phases were combined, with saturated sodium chloride solution (50 mL), dried over anhydrous sodium sulfate, and filtered. The filtrate was concentrated under reduced pressure to give the title product, compound q-4 (0.6 g, 62.5% yield).
MS m/z (ESI): 372.2 [M+1]⁺

### Step 3:

The resin compound S-4 (2.11 g, 0.68 mmol) was swollen with DMF (20 mL) at room temperature for 30 min. Compound q-4 (760 mg, 2.05 mmol), HATU (779 mg, 2.05 mmol), HOBt (277 mg, 2.05 mmol), and DIEA(529 mg, 4.09 mmol) were dissolved in DMF (15 mL), and the solution was then added to the swollen resin. The mixture was left to react at room temperature for 2.5 h. A small amount of resin was collected, filtered under reduced pressure, and then washed with DMF (2 mL × 3). A ninhydrin test of the resin yielded no color change. The reaction resin was filtered under reduced pressure and then washed with DMF (50 mL × 3), DCM (50 mL × 3), and isopropyl ether (50 mL × 3) to give a wet resin compound q-5.

### Step 4:

The resin compound q-5 obtained in the previous step was added to TFA:Tis:H₂O = 95:2.5:2.5 (20 mL) at room temperature, and the mixture was left to react at room temperature for 2 h. The resin was filtered under reduced pressure and with TFA (5 mL × 3). The filtrate was concentrated under reduced pressure until no significant fraction was produced. The resulting oily liquid was added dropwise to isopropyl ether (20 mL). After filtration, the filter cake was purified by preparative chromatography to give compound q (20 mg).
MS m/z (ESI): 573.2 [M+1]⁺.
1H NMR (400 MHz, D2O): δ 8.04 (d, 1H), 7.86 (dd, 3H), 7.65 (d, 1H), 7.49 (d, 2H), 4.18 (s, 2H), 4.18 (s, 2H), 4.12-4.06 (m, 2H), 3.29 (s, 1H), 3.36-3.20 (m, 2H), 2.32 (t, 2H), 2.03-1.94 (m, 1H), 1.87-1.72 (m, 2H), 1.70-1.47 (m, 3H), 1.37 (d, 2H).

### Example 7

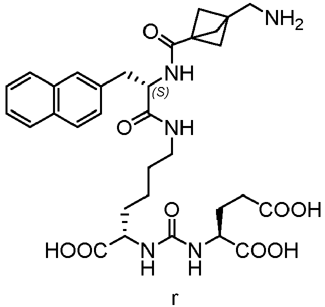

### (((S)-5-((S)-2-(3-(aminomethyl)bicyclo[1.1.1]pentane-1-carboxamido)-3-(naphthalen-2-yl)prop anamido)-1-carboxypentyl)carbamoyl)-L-glutamic acid

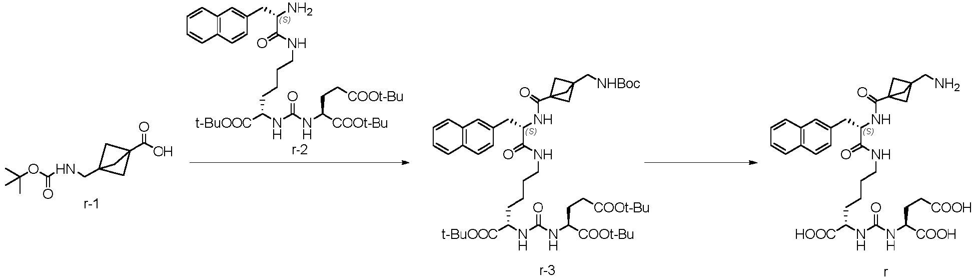

### Step 1:

r-1 (106 mg, 0.44 mmol), HATU (166 mg, 0.44 mmol), DIEA (113 mg, 0.88 mmol), and DCM (10 mL) were added to a reaction flask at room temperature and stirred to form a clear solution, r-2 (200 mg, 0.29 mmol) was added, and the mixture was stirred overnight. TLC monitoring showed the reaction was complete. The reaction solution was washed with water and extracted with DCM (30 mL × 3). The organic phases were combined, washed with 20 mL of saturated sodium chloride solution, dried over anhydrous sodium sulfate, and filtered. The filtrate was concentrated under reduced pressure and purified by silica gel column chromatography eluting with an eluent system of dichloromethane/methanol = methanol (0%-7%) to give r-3 (200 mg, 75.4% yield).
MS m/z (ESI): 908.9 [M+1]⁺

### Step 2:

r-3 (200 mg, 0.42 mmoL) was dissolved in TFA (5 mL) at room temperature. After the addition, the solution was left to react at 33 °C overnight. The reaction solution was concentrated to dryness and purified by high-pressure preparative liquid chromatography to give the title product r (71.5 mg, 50.7% yield).
MS m/z (ESI): 640.4 [M-1]⁻

### Example 8

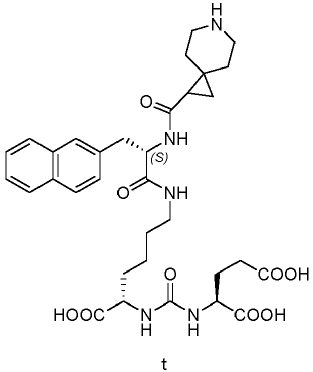

### (((1S)-1-carboxy-5-((2S)-3-(naphthalen-2-yl)-2-(6-azaspiro[2.5]octane-1-carboxamido)propana mido)pentyl)carbamoyl)-L-glutamic acid

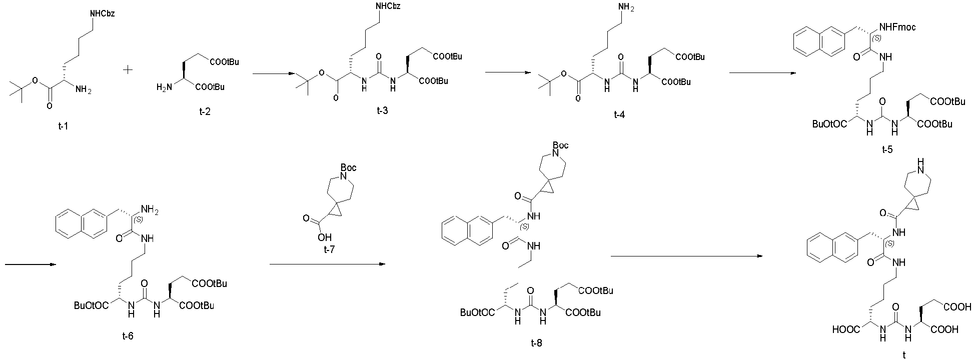

### Step 1:

N-E-Benzyloxycarbonyl-L-lysine tert-butyl ester hydrochloride (10 g, 0.03 mol) and DIEA (3.84 g, 0.03 mol) were dissolved in 120 mL of DCM. The solution was cooled to -10 °C-0 °C and stirred for 0.5 h, and triphosgene (4.4 g, 0.015 mol) was added. After the addition, DIEA (19.2 g, 0.149 mol) was added dropwise at -10 °C-0 °C. After the dropwise addition, the mixture was left to react at that temperature for 3 h. L-Glutamic acid di-tert-butyl ester hydrochloride (10 g, 0.039 mol) was added, and the mixture was naturally warmed to room temperature and stirred overnight. TLC monitoring showed the reaction was complete. The reaction solution was washed with 100 mL of saturated NaHCO₃ solution and then with 100 mL of saturated sodium chloride solution, dried over anhydrous sodium sulfate, and filtered. The filtrate was concentrated under reduced pressure and purified by silica gel column chromatography eluting with an eluent system of n-heptane/ethyl ester = 10:1-1:1. The solvent was evaporated, and t-3 was obtained as an oil (9.8 g, 53.2% yield).
MS m/z (ESI): 622.3 [M+1]⁺

### Step 2:

t-3 (9.8 g, 15.8 mmol) was dissolved in 100 mL of methanol solution at room temperature, and the mixture was stirred to form a clear solution. Pd/C (4.9 g, 58% water content) was added. The reaction flask was purged with nitrogen 3 times and hydrogen 3 times. The mixture was stirred at room temperature for 5 h, and TLC monitoring showed the reaction was complete. The reaction solution was filtered, and the filtrate was concentrated to dryness under reduced pressure to give t-4 (6.2 g, 80.7% yield).
MS m/z (ESI): 488.3 [M+1]⁺

### Step 3:

Fmoc-3-(2-naphthyl)-L-alanine (2.3 g, 5.3 mmol), HATU (2.0 g, 5.3 mmol), DIEA (2.1 g, 16.4 mmol), and DCM (20 mL) were added to a reaction flask at room temperature and stirred to form a clear solution, t-4 (2 g, 4.1 mmol) was added, and the mixture was stirred overnight. TLC monitoring showed the reaction was complete. The reaction solution was washed with water and extracted with EA (30 mL × 3). The organic phases were combined, washed with 50 mL of saturated sodium chloride solution, dried over anhydrous sodium sulfate, and filtered. The filtrate was concentrated under reduced pressure and purified by silica gel column chromatography eluting with an eluent system of dichloromethane/methanol = methanol (0%-10%) to give t-5 (2.8 g, 76% yield).
MS m/z (ESI): 907.5 [M+1]⁺

### Step 4:

t-5 (2.8 g, 3 mmol) and DCM (20 mL) were added to a reaction flask at room temperature and stirred to form a clear solution. Diethylamine was added, and the mixture was stirred overnight. The reaction solution was washed with water and extracted with EA (30 mL × 3). The organic phases were combined, washed with 50 mL of saturated sodium chloride solution, dried over anhydrous sodium sulfate, and filtered. The filtrate was concentrated under reduced pressure and purified by silica gel column chromatography eluting with an eluent system of n-heptane/ethyl ester = EA (0%-100%) to give the title product t-6 (1.4 g, 66.7% yield).
MS m/z (ESI): 685.4 [M+1]⁺

### Step 5:

6-Boc-6-azaspiro[2.5]octane-1-carboxylic acid (145 mg, 0.57 mmol), HATU (216 mg, 0.57 mmoL), DIEA (226 mg, 1.75 mmoL), and DCM (4 mL) were added to a reaction flask at room temperature and stirred to form a clear solution, t-6 (300 mg, 0.44 mmol) was added, and the mixture was stirred overnight. TLC monitoring showed the reaction was complete. The reaction solution was washed with water and extracted with EA(30 mL × 3). The organic phases were combined, washed with 50 mL of saturated sodium chloride solution, dried over anhydrous sodium sulfate, and filtered. The filtrate was concentrated under reduced pressure and purified by silica gel column chromatography eluting with an eluent system of dichloromethane/methanol = methanol (0%-10%) to give the title product t-8 (300 mg, 74.2% yield).
MS m/z (ESI): 922.8 [M+1]⁺

### Step 6:

t-8 (300 mg, 325 mmol) was dissolved in 2 mL of DCM at room temperature, and TFA (3 mL) was added. After the addition, the mixture was left to react at 30 °C overnight. The reaction solution was concentrated to dryness and purified by high-pressure preparative liquid chromatography to give the title product t (70 mg, 32.8% yield).
MS m/z (ESI): 654.3 [M-1]⁻

### Example 9

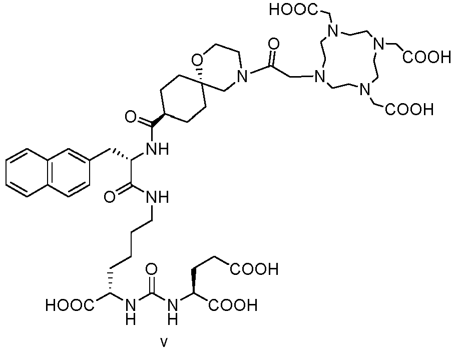

(((S)-1-carboxy-5-((S)-3-(2-naphthyl)-2-((6S,9r)-4-(2-(4,7,10-tris(carboxymethyl)-1,4,7,10-tetraaza cyclododecanyl)acetyl)-1-oxa-4-azaspiro[5.5]undecane-9-carboxamido)propanamido)pentyl)carba moyl)-L-glutamic acid

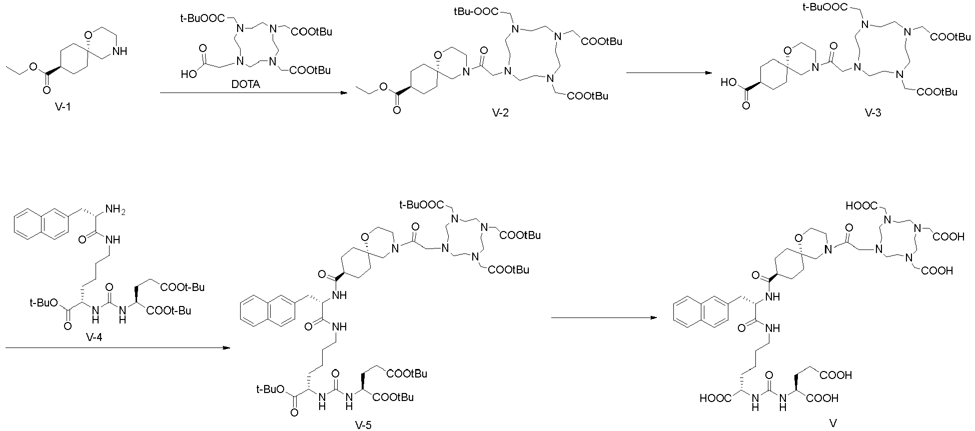

### Step 1:

DOTA (1636 mg, 2.86 mmol), HATU (1087 mg, 2.86 mmol), DIEA (N,N-diisopropylethylamine, 851 mg, 6.6 mmol), and DCM (10 mL) were added to a reaction flask at room temperature and stirred to form a clear solution, v-1 (500 mg, 2.20 mmol) was added, and the mixture was stirred overnight. TLC monitoring showed the reaction was complete. The reaction solution was washed with water and extracted with EA (30 mL × 3). The organic phases were combined, washed with saturated sodium chloride solution, dried over anhydrous sodium sulfate, and filtered. The filtrate was concentrated under reduced pressure and purified by silica gel column chromatography eluting with an eluent system of dichloromethane/methanol to give the title product v-2 (1290 mg, 75.1% yield).
MS m/z (ESI): 782.5 [M+1]⁺

### Step 2:

v-2 (800 mg, 1.01 mmol) was dissolved in 12 mL of THF and 10 mL of water at room temperature, and lithium hydroxide (73 mg, 3.2 mmol) was added. After the addition, the mixture was left to react at room temperature overnight. Dilute hydrochloric acid was added to the reaction solution to adjust the pH to 2-3. The reaction solution was stirred for 0.5 h, and a pH test showed the pH did not change. The reaction solution was extracted with an ethyl ester. The organic phases were combined, washed with saturated brine, dried over anhydrous sodium sulfate, and filtered. The filtrate was concentrated to dryness under reduced pressure to give the title product v-3 (675 mg, 87.5% yield).
MS m/z (ESI): 754.4 [M+1]⁺

### Step 3:

v-3 (600 mg, 0.80 mmol), HATU (303 mg, 0.80 mmol), DIEA (316 mg, 2.45 mmol), and DCM (8 mL) were added to a reaction flask at room temperature and stirred to form a clear solution, v-4 (420 mg, 0.61 mmol, see t-6 of Example 8 for the preparation method) was added, and the mixture was stirred. TLC monitoring showed the reaction was complete. The reaction solution was washed with water and extracted with EA (30 mL × 3). The organic phases were combined, washed with saturated sodium chloride solution, dried over anhydrous sodium sulfate, and filtered. The filtrate was concentrated under reduced pressure and purified by silica gel column chromatography eluting with an eluent system of dichloromethane/methanol to give v-5 (638 mg, 73.3% yield).
MS m/z (ESI): 1421 [M+1]⁺

### Step 4:

v-5 (500 mg, 0.35 mmol) was dissolved in 4 mL of DCM at room temperature, and TFA (5 mL) was added. After the addition, the mixture was left to react at 30 °C overnight. The reaction solution was concentrated to dryness and purified by high-pressure preparative liquid chromatography to give the title product v (27 mg, 7.1% yield).
MS m/z (ESI): 1084.5 [M-1]⁻
1H NMR (400 MHz, Deuterium Oxide) δ 7.84(t, 3H), 7.65(s, 1H), 7.47-7.49 (m, 2H), 7.38 (d, 1H), 4.55 (t, 1H), 4.18-4.19 (m, 1H), 3.10-3.87 (m, 33H), 2.42(t, 2H),1.91 (dt, 1H), 1.89 (m, 1H), 1.87 (m, 3H), 0.84-1.42 (m, 14H).

### Example 10

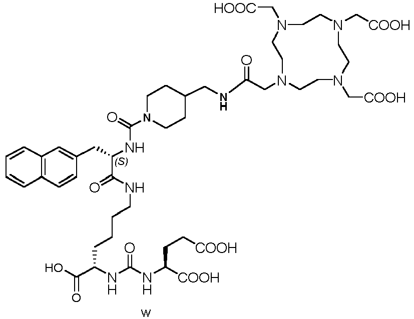

(((S)-1-carboxy-5-((S)-3-(2-naphthyl)-2-(4-((2-(4,7,10-tris(carboxymethyl)-1,4,7,10-tetraazacyclod odecanyl)-1-acetyl)methyl)piperidine-1-carboxamido)propanamido)pentyl)carbamoyl)-L-glutamic acid

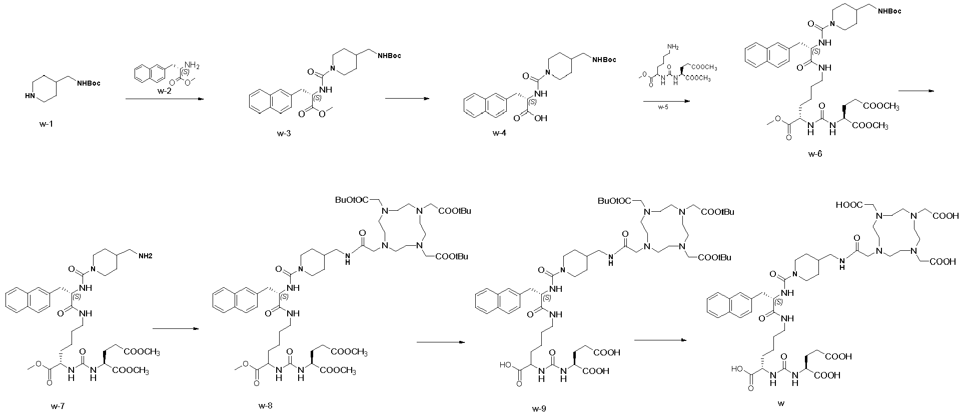

### Step 1:

w-2 (3 g, 11.3 mmol) and DIEA (1.45 g, 11.3 mmol) were dissolved in 120 mL of DCM at room temperature. The solution was cooled to -10 °C-0 °C and stirred for 0.5 h, and triphosgene (1.7 g, 5.7 mmol) was added. After the addition, DIEA (7.3 g, 56.6 mmol) was added dropwise at -10 °C-0 °C. After the dropwise addition, the mixture was left to react at that temperature for 3 h. w-1 (3.2 g, 14.7 mmol) was added, and the mixture was naturally warmed to room temperature and stirred overnight. TLC monitoring showed the reaction was complete. The reaction solution was washed with 100 mL of saturated NaHCO₃ solution and then with 100 mL of saturated sodium chloride solution, dried over anhydrous sodium sulfate, and filtered. The filtrate was concentrated under reduced pressure and purified by silica gel column chromatography eluting with an eluent system of n-heptane/ethyl ester = 10:1-1:1. The solvent was evaporated, and w-3 was obtained as a white solid (4.0 g, 75.5% yield).
MS m/z (ESI): 470.6 [M+1]⁺

### Step 2:

w-3 (4.0 g, 8.5 mmol) was dissolved in 20 mL of THF and 10 mL of water at room temperature, and lithium hydroxide (0.62 g, 25.6 mmol) was added. After the addition, the mixture was left to react at room temperature overnight. 10 mL of water was added to the reaction solution, and the mixture was extracted with ethyl acetate (10 mL × 2). The aqueous phases were combined and cooled to 0 °C in an ice bath, and the pH was adjusted to 3-4 with 0.5 N citric acid. A solid precipitated. The mixture was stirred for 0.5 h, and a pH test showed the pH did not change. The mixture was filtered, and the filter cake was dried *in vacuo* (40 °C, 4 h) to constant weight to give the title product w-4 (3.4 g, 87.6% yield).
MS m/z (ESI): 456.6 [M+1]⁺

### Step 3:

w-4 (1.0 g, 2.2 mmol), HATU (1.02 g, 2.7 mmol), DIEA (1.39 g, 10.8 mmol), and DCM (20 mL) were added to a reaction flask at room temperature and stirred to form a clear solution, w-5 (0.66 g, 1.8 mmol) was added, and the mixture was stirred overnight. The reaction solution was washed with water and extracted with DCM (40 mL × 3). The organic phases were combined, washed with 50 mL of saturated sodium chloride solution, dried over anhydrous sodium sulfate, and filtered. The filtrate was concentrated under reduced pressure and purified by silica gel column chromatography eluting with an eluent system of dichloromethane/methanol = methanol (0%-10%) to give the title product w-6 (0.86 g, 59.4% yield).
MS m/z (ESI): 799.4 [M+1]⁺

### Step 4:

w-6 (0.86 g, 1.1 mmol) was dissolved in 2 mL of ethyl acetate at room temperature, and a 4 M HCl/EA solution (8 mL, 32 mmol) was added with stirring. The mixture was stirred for 2 h. TLC monitoring showed the reaction was complete. The reaction solution was concentrated under reduced pressure to constant weight to give the title product w-7 (0.82 g, 95.3% yield).
MS m/z (ESI): 699.3 [M+1]⁺

### Step 5:

DOTA-tris(t-Bu ester) (209 mg, 0.36 mmol), HATU (137 mg, 0.36 mmol), DIEA (464 mg, 3.6 mmol), and DCM (3 mL) were added to a reaction flask at room temperature and stirred to form a clear solution, w-7 (170 mg, 0.24 mmol) was added, and the mixture was stirred overnight. There was starting material left. More DOTA-tris(t-Bu ester) (139 mg, 0.24 mmol) and HATU (91 mg, 0.24 mmol) were added. After another 2 h, the reaction was complete. The reaction solution was washed with water and extracted with DCM (20 mL × 3). The organic phases were combined, washed with 20 mL of saturated sodium chloride solution, dried over anhydrous sodium sulfate, and filtered. The filtrate was concentrated under reduced pressure and purified by silica gel column chromatography eluting with an eluent system of dichloromethane/methanol = methanol (0%-20%) to give w-8 (110 mg, 36.6% yield).
MS m/z (ESI): 1253.4 [M+1]⁺

### Step 6:

w-8 (110 mg) was dissolved in 2 mL of THF and 1 mL of water at room temperature, and lithium hydroxide was added. After the addition, the mixture was left to react at room temperature for 2 h. After the reaction was complete, the reaction solution was concentrated to dryness under reduced pressure to give w-9.

### Step 7:

w-9 and TFA (2 mL) were added to a reaction flask at room temperature and stirred to form a clear solution. After the addition, the solution was left to react at room temperature for 2 h. After the reaction was complete, the reaction solution was concentrated to dryness under reduced pressure and purified by high-pressure preparative liquid chromatography to give the title product w (13 mg).
MS m/z (ESI): 1043.6 [M+1]⁺

### Example 11.

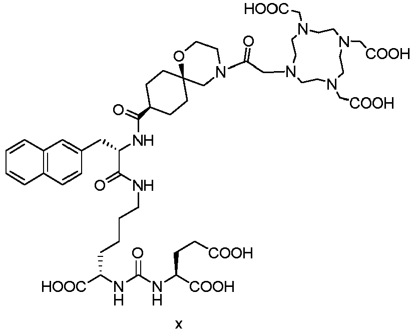

(((S)-1-carboxy-5-((S)-3-(2-naphthyl)-2-((6R,9s)-4-(2-(4,7,10-tris(carboxymethyl)-1,4,7,10-tetraaza cyclododecanyl)acetyl)-1-oxa-4-azaspiro[5.5]undecane-9-carboxamido)propanamido)pentyl)carba moyl)-L-glutamic acid

Compound x was obtained using the preparation method of Example 9.

### Example 12. Preparation of Compound ¹⁷⁷Lu-v

The total volume of the reaction was 400 µL, including 15 nmol of compound v and 15 mCi of ¹⁷⁷Lu. To a 1.5 mL centrifuge tube was added 321 µL of acetic acid-sodium acetate buffer (0.1 M, pH 4.5), followed by 15 µL of a solution of compound v. 7 µL of the nuclide ¹⁷⁷LuCl₃ (activity: 15.23 mCi) was taken. The reaction was shaken on a thermostatic mixer at 95 °C, and the reaction time was 15 min. Activity: 15.15 mCi. The HPLC result reached >99%.

### Example 13. Preparation of Compound ⁶⁸Ga-v

13.5 mg of compound v was weighed out and dissolved in ultrapure water to form a 25 mL solution. 136 mg of sodium acetate trihydrate was weighed out and dissolved in 1 mL of ultrapure water. 20 µL of the solution obtained in step 1 was transferred to a reaction vial using a pipette, and 4.5 mL of a hydrochloric acid eluate of ⁶⁸GaCl₃ and 0.5 mL of the buffer of step 2 were added sequentially. The vial was gently shaken to mix the contents, left to stand at 95 °C for 10 min, and naturally cooled to room temperature. The reaction mixture was sent for analysis and used.

### Test Example 1. Tests for Inhibitory Activity Against PSMA

### I. Experimental materials and instruments

1. Multifunctional microplate reader (SPARK, TECAN)
2. rhPSMA (R&D, 4234-ZN)
3. N-Acetyl-Asp-Glu (Sigma, A5930)
4. OPA (Sigma, P0657)

### II. Experimental procedure

PSMA inhibitors can bind to the enzyme PSMA to prevent the enzyme PSMA from decomposing the substrate N-Acetyl-Asp-Glu. In this experiment, the extent to which the substrate was decomposed and the resulting ultraviolet absorption changes were measured to evaluate the capacities of the PSMA inhibitors to bind to the enzyme PSMA, and the activity of the compounds was evaluated with IC₅₀ values.

Buffer 1 (50 mM HEPES, 0.1 M NaCl, pH 7.5) was used to prepare a 0.4 µg/mL rhPSMA solution and a 40 µM solution of the substrate N-Acetyl-Asp-Glu. rhPSMA was mixed with the small molecules to be tested in a 96-well plate, with a constant rhPSMA content of 50 ng/well maintained. Meanwhile, the small molecules were step-wise diluted to final concentrations of 1 µM, 100 nM, 33.3 nM, 11.1 nM, 3.7 nM, 1.2 nM, 0.41 nM, 0.137 nM, 0.045 nM, and 0 nM. In addition, a positive control was set up using PSMA-617. The rhPSMA-small molecules were taken at 40 µL/well and well mixed with the 40 µM solution of the substrate N-Acetyl-Asp-Glu (40 µL/well). The mixtures were incubated at 37 °C in the dark for 1 h, were heated at 70 °C for 5 min to quench the reactions, and were cooled to room temperature. Buffer 2 (0.2 M NaOH, 0.1% beta-Mercaptoethanol) was used to prepare a 15 mM OPA solution. The OPA solution was added to the reaction systems at 80 µL/well and well mixed, and then the plate was incubated at room temperature for 10 min. The mixtures were taken at 100 µL/well and added to a 96-well Flat Black. With the excitation wavelength set to 330 nm and the emission wavelength to 465 nm, the intensity of signals was measured. IC₅₀ values were obtained from dose-response curves.

### III. Experimental data

The capacities of the compounds of the present disclosure to bind to the enzyme GCPII can be measured through the assay described above. The IC₅₀ measurements are shown in Table 1.

**Table 1. The IC₅₀ values of compounds**

| Compound structure | IC₅₀ (nM) |
|---|---|
| 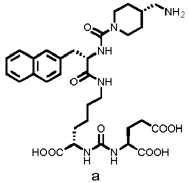 | 6.232 |
| 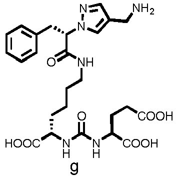 | 142.6 |
| 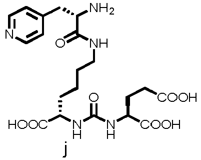 | 177.5 |
| 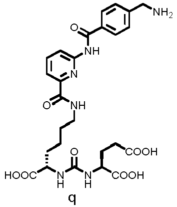 | 2.422 |
| 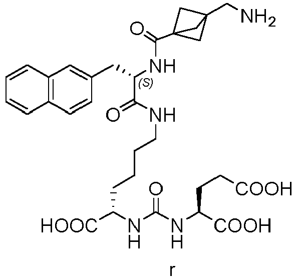 | 2.833 |
| 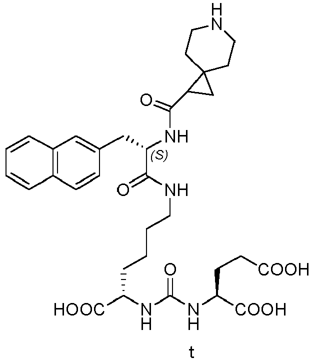 | 2.396 |
| 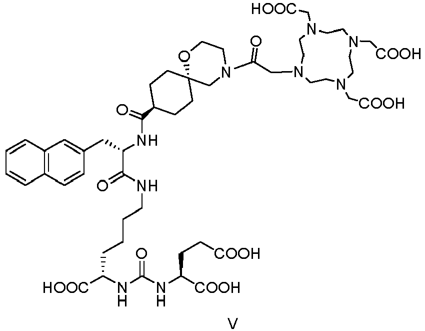 | 1.518 |
| 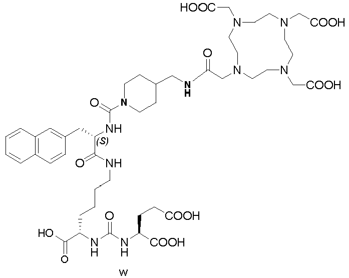 | 4.135 |
| PSMA-617 | 2.358 |

### Test Example 2. Affinity Assays Using Enzyme Activity Method

Buffer 1 (50 mM HEPES, 0.1 M NaCl, pH 7.5) was used to prepare a 0.4 µg/mL rhPSMA solution and a 40 µM solution of the substrate N-Acetyl-Asp-Glu. rhPSMA was mixed with the small molecules to be tested in a 96-well plate, with a constant rhPSMA content of 50 ng/well maintained. Meanwhile, the small molecules were step-wise diluted to final concentrations of 1 µM, 100 nM, 33.3 nM, 11.1 nM, 3.7 nM, 1.2 nM, 0.41 nM, 0.137 nM, 0.045 nM, and 0 nM. In addition, a positive control was set up using PSMA-617. The rhPSMA-small molecules were taken at 40 µL/well and well mixed with the 40 µM solution of the substrate N-Acetyl-Asp-Glu (40 µL/well). The mixtures were incubated at 37 °C in the dark for 1 h, were heated at 70 °C for 5 min to quench the reactions, and were cooled to room temperature. Buffer 2 (0.2 M NaOH, 0.1% beta-Mercaptoethanol) was used to prepare a 15 mM OPA solution. The OPA solution was added to the reaction systems at 80 µL/well and well mixed, and then the plate was incubated at room temperature for 10 min. The mixtures were taken at 100 µL/well and added to a 96-well Flat Black. With the excitation wavelength set to 330 nm and the emission wavelength to 465 nm, the intensity of signals was measured. IC₅₀ values were obtained from dose-response curves.

**Table 2.**

| Compound | IC₅₀ (nM) | Ratio to PSMA-617 |
|---|---|---|
| PSMA-617 | 1.654 | 1.00 |
| Example 9 | 1.231 | 0.74 |
| Example 11 | 13.01 | 7.85 |

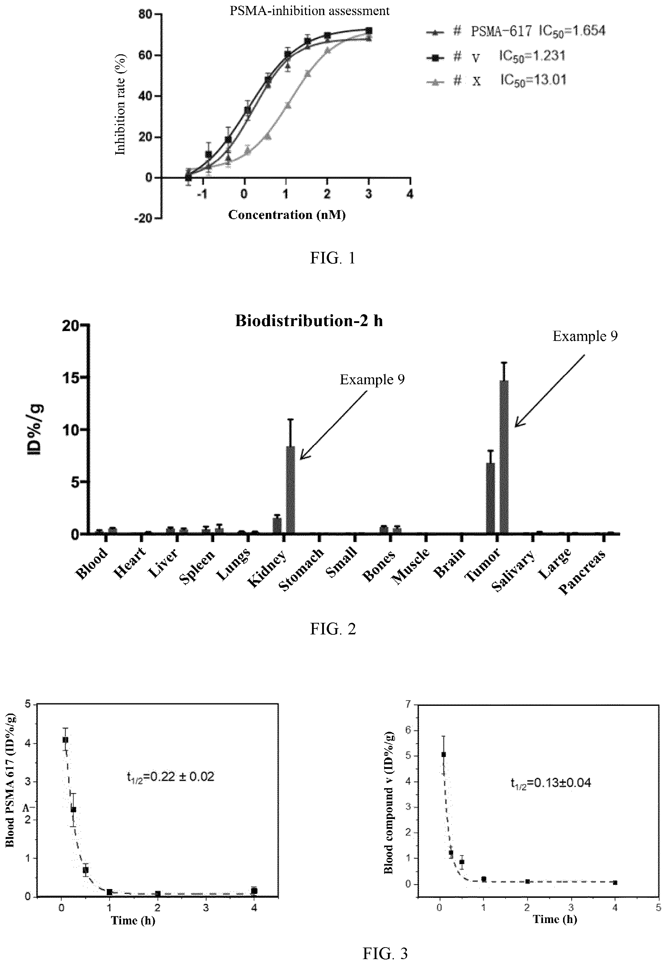

The specific structure is shown in FIG. 1. Through the enzyme activity experiment, it can be determined that compound v of Example 9 has a better affinity than compound x of Example 11.

### Test Example 3. Biodistribution of Compounds in Tumor-Bearing Mice

After single tail-vein injections into mice, the *in-vivo* distributions of ⁶⁸Ga-labeled compound v (Example 9) and compound x (Example 11) in the positive LnCaP tumor-bearing mice were observed.

At 2 h after the injection, a total of 3 animals were sacrificed by cervical dislocation, and tissue samples were collected, including samples of the blood, the heart, the lungs, the liver, the spleen, the kidneys, the stomach, the intestine, the bones, the flesh, the brain, the salivary gland, the large intestine, the pancreas, and the tumor. The net weight of the tissues was measured first, and the radioactivity counts of the collected tissues were then measured using a γ-counter. The distributions of the labeled compounds in the different tissues and organs of the mice were measured. Meanwhile, a test sample was accurately diluted 100-fold, and 0.1 mL of the dilution was added to a counting tube and used as standard 1% ID (i.e., one percent of the injected dose). The radioactivity counts of the 1% ID standard and the biological samples were measured simultaneously on a γ-counter. The biodistribution data were expressed as the percentage of the radioactivity counts per gram of tissue or organ to the total injected dose (radioactivity counts) (% ID/g).

The specific results are shown in FIG. 2, and the results show that the uptake value of ⁶⁸Ga-v (Example 9) in the LnCap tumor is the greatest and is ~10 Id%/g, followed by those in the kidneys, the liver, the lung, and the spleen, and the uptake values in the other tissues are all very low. The comparison shows that ⁶⁸Ga-v (Example 9) has a good targeting effect on the LnCap tumor. ⁶⁸Ga-v (Example 9)'s tumor uptake is better than ⁶⁸Ga-x (Example 11)'s.

### Test Example 4. Pharmacokinetics and Toxicity

### 4.1. Half-life of ⁶⁸Ga-v (Example 9) in blood

Through single tail-vein injections into mice, the pharmacokinetics of ⁶⁸Ga-v (Example 9) and PSMA-617 in blood was studied.

Each mouse was dosed at 50 µCi/100 µL, and blood samples were taken from the orbit 0.083, 0.25, 0.5, 1, 2, and 4 h post-dose (4 animals per time point). The blood samples were collected in pre-weighed sample tubes. The tubes were weighed and the weight of the blood samples was recorded. Then radioactivity counting was performed using a γ-counter. Meanwhile, a test sample was accurately diluted 100-fold, and 0.1 mL of the dilution was added to a counting tube and used as standard 1% ID (i.e., one percent of the injected dose). The radioactivity counts of the 1% ID standard and the biological samples were measured simultaneously on a γ-counter. The data on blood were expressed as the percentage of the radioactivity counts per gram of blood to the total injected dose (radioactivity counts) (% ID/g). Pharmacokinetic parameters were calculated from blood drug concentration data.

The uptake results in the blood of normal mice are shown in Table 3 below (n = 4).

**Table 3.**

| Blood %ID/g Time/h | ⁶⁸Ga-v (Example 9) | | | | ⁶⁸Ga-PSMA-617 | | | |
|---|---|---|---|---|---|---|---|---|
| | 1 | 2 | 3 | 4 | 1 | 2 | 3 | 4 |
| 0.083 | 5.89 | 4.98 | 5.26 | 4.11 | 4.22 | 4.29 | 3.66 | 4.22 |
| 0.25 | 1.33 | 1.11 | 1.52 | 1.03 | 2.12 | 2.53 | 2.71 | 1.73 |
| 0.5 | 1.15 | 0.52 | 0.79 | 0.99 | 0.55 | 0.84 | 0.86 | 0.55 |
| 1 | 0.33 | 0.19 | 0.19 | 0.16 | 0.14 | 0.16 | 0.10 | 0.13 |
| 2 | 0.07 | 0.13 | 0.10 | 0.11 | 0.04 | 0.07 | 0.10 | 0.13 |
| 4 | 0.06 | 0.02 | 0.10 | 0.08 | 0.29 | 0.10 | / | 0.09 |

The calculated pharmacokinetic parameters are shown in Table 4 below (0-4 h).

**Table 4.**

| Pharmacokinetic parameters | Unit | ⁶⁸Ga-V (Example 9) | ⁶⁸Ga-PSMA-617 |
|---|---|---|---|
| t_{1/2} | h | 0.13±0.04 | 0.22±0.02 |
| Cₘₐₓ | %ID/g | 5.06±0.74 | 4.10±0.29 |
| AUCₗₐₛₜ | h*%ID/g | 1.67±0.26 | 1.66±0.14 |
| AUC_{INF_obs} | h*%ID/g | 1.66±0.24 | 1.65±0.13 |
| Vz_obs | µCi/%ID/g | 4.97±1.09 | 4.06±1.39 |
| Cl_obs | µCi/h*%ID/g | 12.17±1.77 | 12.10±1.0 |
| MRTₗₐₛₜ | h | 0.39±0.02 | 0.33±0.05 |

The above experimental results show that after ⁶⁸Ga-v (Example 9) and ⁶⁸Ga-PSMA-617 entered the blood of normal mice, the radioactive substance was rapidly distributed; 0.25 h after the injections, the contents of the radioactive substance in the blood were only 1.25 ± 0.22% ID/g and 2.27 ± 0.44% ID/g; the radioactive substance was rapidly cleared from the blood, and the half-lives in the blood were only 0.13 h (7.8 min) and 0.22 h (13.2 min).

⁶⁸Ga-v (Example 9) has a half-life of 0.13 h (7.8 min) in the blood of mice and an elimination phase half-life of 0.758 h. Generally, the time it takes to complete the metabolism is estimated to be 5 times the half-life; therefore, ⁶⁸Ga-v should be substantially metabolized 3.9 h after dosing. In addition, according to imaging data, few signals were detected from the normal organs after 4 h. According to calculations, the effective half-life is Te = 0.45 h; it is 2.27 h, as estimated using 5 effective half-lives.

### 4.2. Absorbed doses of radiation

The AUC of drug metabolisms was calculated from the biodistribution (Bio-D) data and imported into the OLINDA software to generate doses of radiation absorbed by all the organs.

**Table 5. A summary of radiation dose estimates for human organs**

| mSv/MBq | | | | |
|---|---|---|---|---|
| Target Organ | ⁶⁸Ga-V (Example 9) | ⁶⁸Ga-PSMA-617 | ¹⁷⁷Lu-PSMA-617 | ¹⁷⁷Lu-V (Example 9) |
| Adrenals | 8.50E-03 | 7.49E-02 | 2.43E-02 | 1.74E-02 |
| Brain | 3.99E-02 | 7.80E-02 | 1.38E-01 | 6.03E-02 |
| Esophagus | 3.77E-03 | 1.79E-02 | 3.39E-03 | 4.33E-03 |
| Eyes | 1.84E-03 | 1.25E-02 | 2.74E-03 | 2.21E-03 |
| Gallbladder Wall | 5.52E-03 | 3.22E-02 | 3.58E-03 | 4.67E-03 |
| Left colon | 6.87E-03 | 1.63E-02 | 4.60E-03 | 4.81E-03 |
| Small Intestine | 1.31E-01 | 1.00E-01 | 4.48E-02 | 1.73E-01 |
| Stomach Wall | 8.54E-02 | 1.91E-02 | 8.22E-03 | 2.73E-02 |
| Right colon | 3.43E-03 | 1.34E-02 | 2.74E-03 | 3.03E-03 |
| Rectum | 1.99E-01 | 1.83E-01 | 1.49E-03 | 1.91E-03 |
| Heart Wall | 1.62E-02 | 3.93E-02 | 2.41E-02 | 2.72E-02 |
| Kidneys | 3.54E-02 | 5.47E-01 | 1.47E+00 | 8.83E-01 |
| Liver | 2.50E-02 | 1.75E-01 | 3.15E-02 | 7.43E-02 |
| Lungs | 1.05E-02 | 1.21E-01 | 1.12E-01 | 1.95E-01 |
| Pancreas | 9.51E-03 | 1.86E-02 | 4.36E-03 | 5.14E-03 |
| Prostate | 3.77E-03 | 7.73E-03 | 1.23E-03 | 1.29E-03 |
| Salivary Glands | 1.81E-03 | 9.39E-03 | 2.18E-03 | 1.67E-03 |
| Red Marrow | 2.12E-03 | 2.11E-02 | 3.80E-03 | 4.16E-03 |
| Osteogenic Cells | 4.65E-03 | 3.14E-01 | 5.58E-01 | 6.52E-01 |
| Spleen | 5.13E-02 | 1.79E-01 | 9.86E-01 | 6.84E-01 |
| Testes | 3.51E-04 | 3.69E-03 | 5.04E-04 | 5.84E-04 |
| Thymus | 1.99E-03 | 1.16E-02 | 2.04E-03 | 2.83E-03 |
| Thyroid | 9.11E-04 | 1.08E-02 | 1.83E-03 | 2.41E-03 |
| Urinary Bladder Wall | 2.27E-03 | 5.54E-03 | 7.53E-04 | 9.14E-04 |
| Total Body | 0.496E-02 | 3.61E-02 | 2.91E-02 | 3.07E-02 |
| Effective Dose | 2.10E-02 | 4.68E-02 | 4.74E-02 | 5.46E-02 |

As can be seen from the table above, the absorbed dose of radiation resulting from ⁶⁸Ga-v (Example 9) is half of that resulting from ⁶⁸Ga-PSMA-617, and thus the former is safer; the absorbed dose of radiation resulting from ¹⁷⁷Lu-v (Example 9) is smaller than that resulting from ¹⁷⁷Lu-PSMA-617.

## Claims

1. A compound represented by formula (IV) or a pharmaceutically acceptable salt thereof, 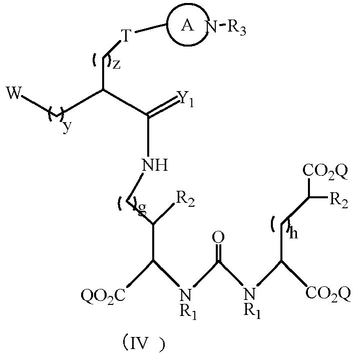 wherein:
Q is selected from the group consisting of H and a protecting group, preferably from H;
R₁ and R₂ are each independently selected from the group consisting of H and C₁₋₄ alkyl and are preferably both H; the C₁₋₄ alkyl is optionally substituted with one or more substituents P or is unsubstituted;
each occurrence of Q, R₁, and R₂ may be the same or different;
Y₁ is S or O, preferably O;
T is selected from the group consisting of -NR₄(CO)-, -NR₄ (SO₂)-, and -NR₄ (CH₂)-;
R₄ is selected from the group consisting of H, C₁-₆ alkyl, 6-10 membered aryl, and 5-12 membered heteroaryl; the C₁-₆ alkyl, 6-10 membered aryl, or 5-12 membered heteroaryl is optionally substituted with one or more substituents P or is unsubstituted;
ring A is selected from 3-12 membered nitrogen-containing heterocyclyl, wherein the 3-12 membered nitrogen-containing heterocyclyl is optionally substituted with one or more substituents P or is unsubstituted;
W is selected from the group consisting of 6-10 membered aryl and 5-12 membered heteroaryl; the -10 membered aryl or 5-12 membered heteroaryl is optionally substituted with one or more substituents P or is unsubstituted;
the substituents P are selected from the group consisting of C₁-C₆ alkyl, halogen, deuterium, hydroxy, sulfhydryl, -NRᵢRⱼ, oxo, thio, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, 5- to 10-membered heteroaryl, 8- to 12-membered fused cycloaryl, and 5- to 12-membered fused heteroaryl;
Rᵢ and Rⱼ are each independently selected from the group consisting of a hydrogen atom, hydroxy, C₁-C₆ alkyl, and C₁-C₆ alkoxy; Rₖ is independently selected from the group consisting of a hydrogen atom, C₁-C₆ alkyl, C₁-C₆ haloalkyl, C₁-C₆ alkoxy, hydroxy, and -NRᵢRⱼ, wherein the alkyl, alkoxy, or haloalkyl is optionally substituted with one or more substituents selected from the group consisting of C₁-C₆ alkyl, halogen, hydrogen, sulfhydryl, -NRᵢRⱼ, oxo, thio, carboxyl, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, and 5- to 10-membered heteroaryl;
y, z, g, and h are each independently integers of 0-6;
R₃ is selected from the group consisting of H and a chelating agent.

2. The compound represented by formula (IV) or the pharmaceutically acceptable salt thereof according to claim 1, wherein T is -NH(CO)-; ring A is a 5-12 membered nitrogen-containing spiroheterocyclyl group, preferably a 5-12 membered nitrogen-containing monospiroheterocyclyl group, more preferably a 3-membered/4-membered, 3-membered/5-membered, 3-membered/6-membered, 4-membered/4-membered, 4-membered/5-membered, 4-membered/6-membered, 5-membered/5-membered, 5-membered/6-membered, or 6-membered/6-membered nitrogen-containing monospiroheterocyclyl group, most preferably 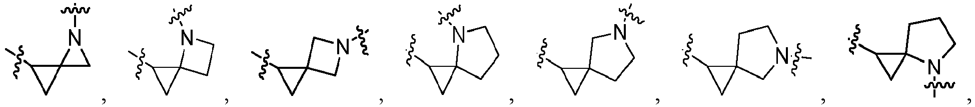 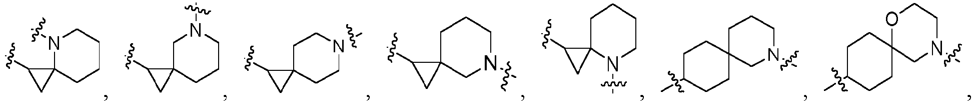 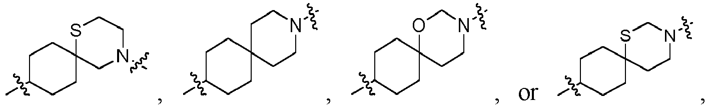 and particularly preferably

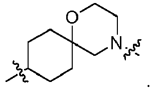

3. The compound represented by formula (IV) or the pharmaceutically acceptable salt thereof according to any of claims 1 to 2, wherein W is selected from 6-10 membered aryl, preferably naphthyl.

4. The compound represented by formula (IV) or the pharmaceutically acceptable salt thereof according to any of claims 1 to 3, wherein Yi is O.

5. The compound represented by formula (IV) or the pharmaceutically acceptable salt thereof according to any of claims 1 to 4, wherein R₁ and R₂ are each independently H.

6. The compound represented by formula (IV) or the pharmaceutically acceptable salt thereof according to any of claims 1 to 5, wherein Q is selected from the group consisting of H and a protecting group, preferably from H.

7. The compound represented by formula (IV) or the pharmaceutically acceptable salt thereof according to any of claims 1 to 6, wherein y and h are each independently selected from the group consisting of 0, 1, and 2, preferably from 1.

8. The compound represented by formula (IV) or the pharmaceutically acceptable salt thereof according to any of claims 1 to 6, wherein g is selected from the group consisting of 3 and 4, preferably from 3.

9. The compound represented by formula (IV) or the pharmaceutically acceptable salt thereof according to any of claims 1 to 6, wherein z is selected from the group consisting of 0 and 1, preferably from 0.

10. The compounds or the pharmaceutically acceptable salts thereof according to claims 1 to 9, wherein the chelating agent is selected from the group consisting of: 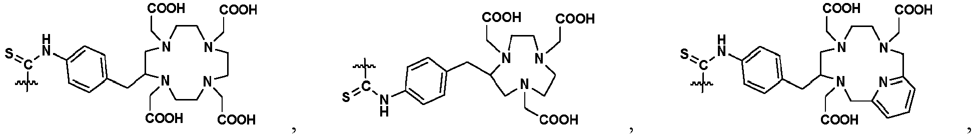 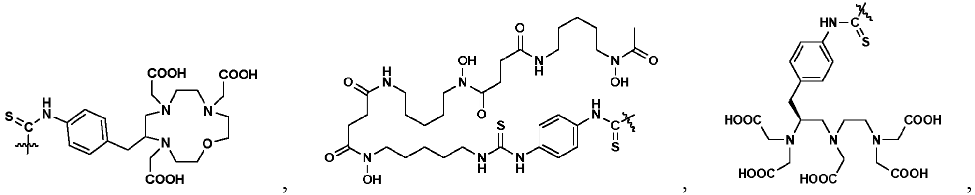 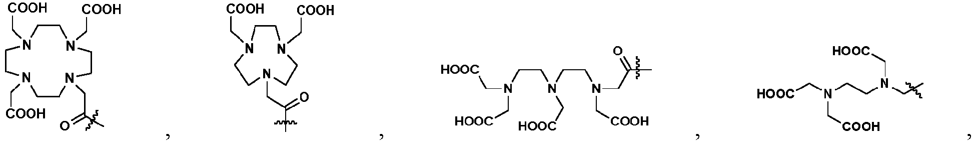 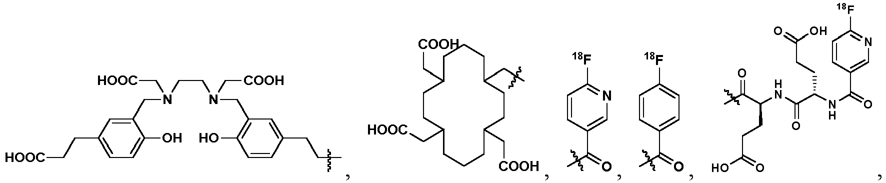 and 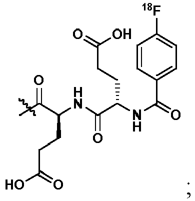 preferably from

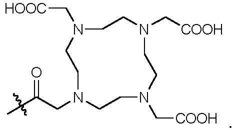

11. The compounds according to claims 1 to 10, being selected from the group consisting of: 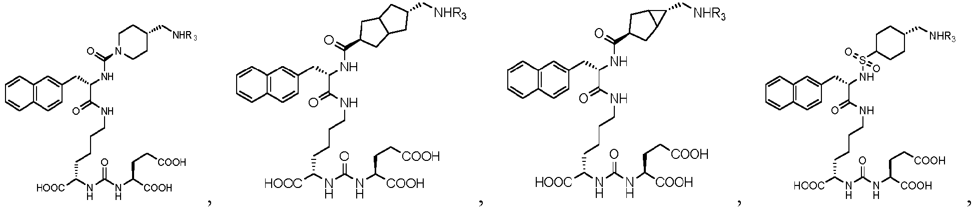 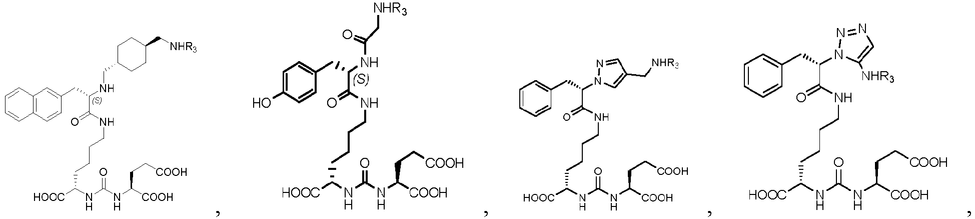 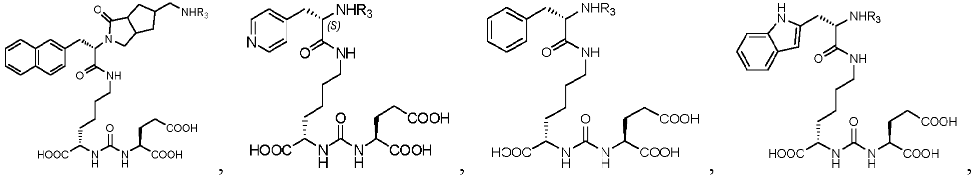 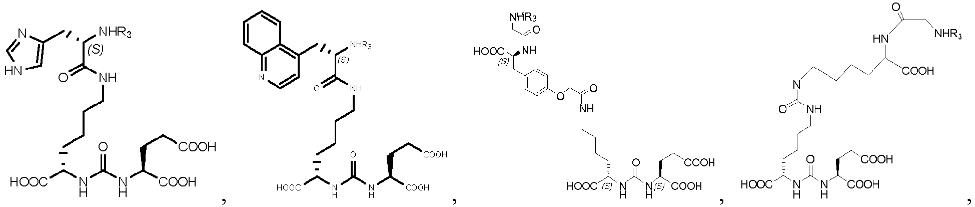 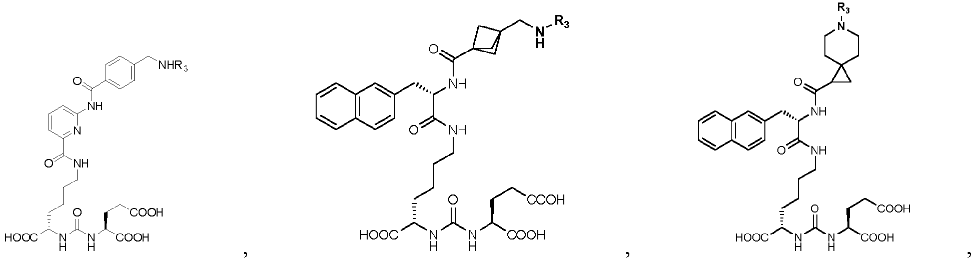 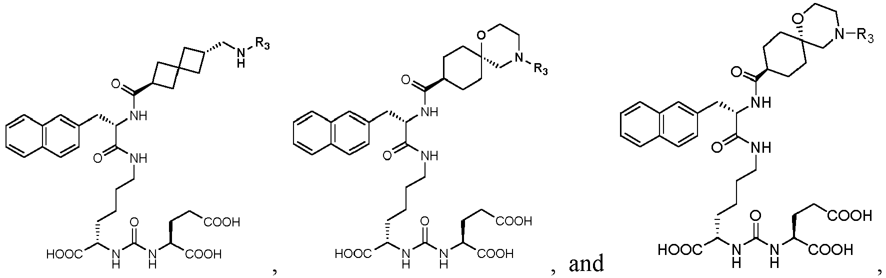 wherein R₃ is selected from the group consisting of H and

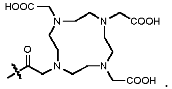

12. The compound represented by formula (IV) or the pharmaceutically acceptable salt thereof according to any of claims 1 to 11, being 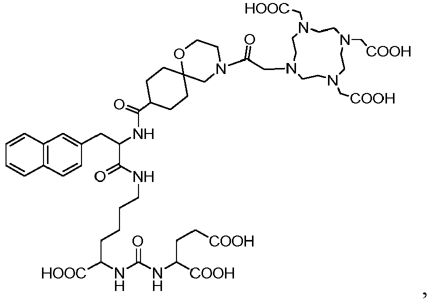 preferably 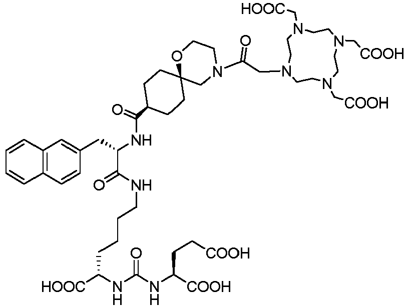 or 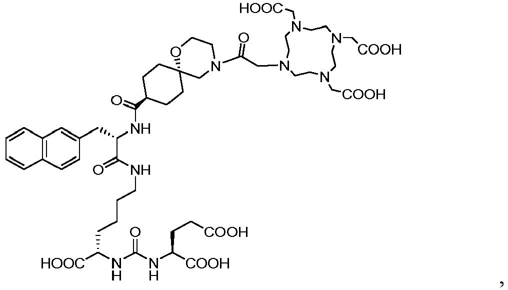 and most preferably

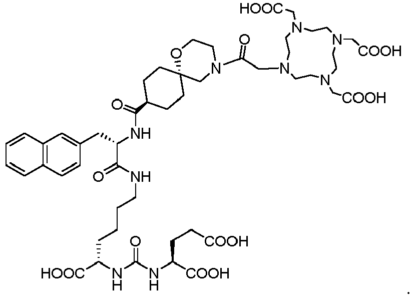

13. The compound according to any of claims 1 to 12, wherein the chelating agent comprises a radionuclide.

14. The compound or the pharmaceutically acceptable salt thereof according to claim 13, wherein the radionuclide is selected from at least one of ¹⁸F, ¹¹C, ⁶⁸Ga, ¹²⁴I, ⁸⁹Zr, ⁶⁴Cu, ⁸⁶Y, ^{99m}Tc, ¹¹¹In, ¹²³I, ⁹⁰Y, ¹²⁵I, ¹³¹I, ¹⁷⁷Lu, ²¹¹At, ¹⁵³Sm, ¹⁸⁶Re, ¹⁸⁸Re, ⁶⁷Cu, ²¹²Pb, ²²⁵Ac, ²¹³Bi, ²¹²Bi, ²¹²Pb, and ⁶⁷Ga, preferably from the group consisting of ⁶⁸Ga and ¹⁷⁷Lu.

15. A compound represented by formula (IV) or a pharmaceutically acceptable salt thereof, being 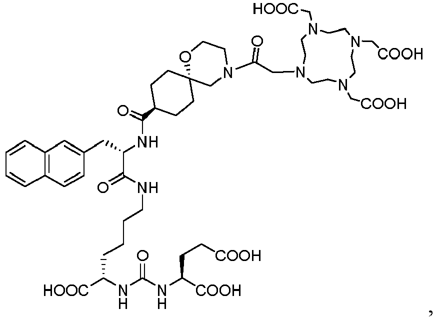 wherein the chelating agent comprises a radionuclide, and the radionuclide is ⁶⁸Ga.

16. A compound represented by formula (IV) or a pharmaceutically acceptable salt thereof, being 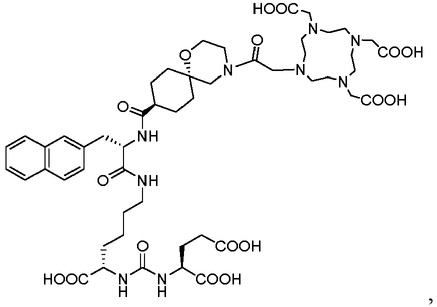 wherein the chelating agent comprises a radionuclide, and the radionuclide is ¹⁷⁷Lu.

17. A pharmaceutical composition, comprising the compound or the pharmaceutically acceptable salt thereof according to any of claims 1 to 16, and one or more pharmaceutically acceptable excipients, diluents, or carriers.

18. Use of the compound or the pharmaceutically acceptable salt thereof according to any of claims 1 to 16 or the composition according to claim 17 in the preparation of a composition for imaging in patients.

19. Use of the compound or the pharmaceutically acceptable salt thereof according to any of claims 1 to 16 or the composition according to claim 17 in the preparation of a medicament for diagnosing and/or treating and/or preventing PSMA-mediated diseases or disorders.

20. Use of the compound or the pharmaceutically acceptable salt thereof according to any of claims 1 to 16 or the composition according to claim 17 in the preparation of a medicament for diagnosing and/or treating and/or preventing a tumor and a cancer, wherein preferably, the tumor and the cancer are prostate cancer.

21. A preparation method for a compound represented by formula (IV) or a pharmaceutically acceptable salt thereof, wherein the compound represented by formula (IV) is the compound represented by formula v or a pharmaceutically acceptable salt thereof; the preparation method comprises the step of removing tert-butyl groups from the compound represented by formula v-5:

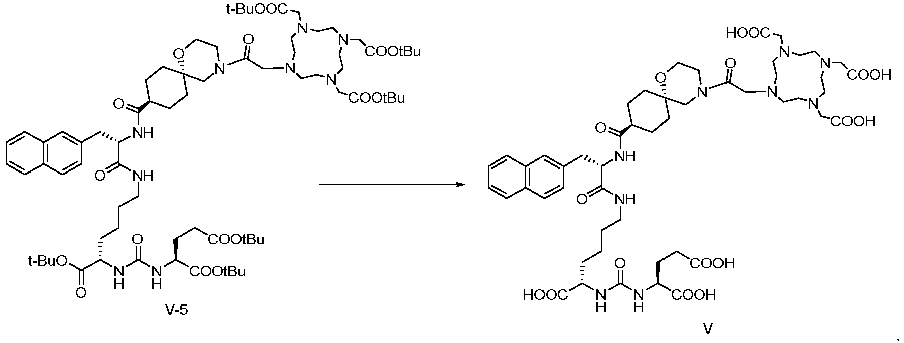

22. The preparation method according to claim 21, further comprising the step of conducting a condensation reaction of the compound represented by formula v-3 with the compound represented by formula v-4 to give the compound represented by formula v-5,

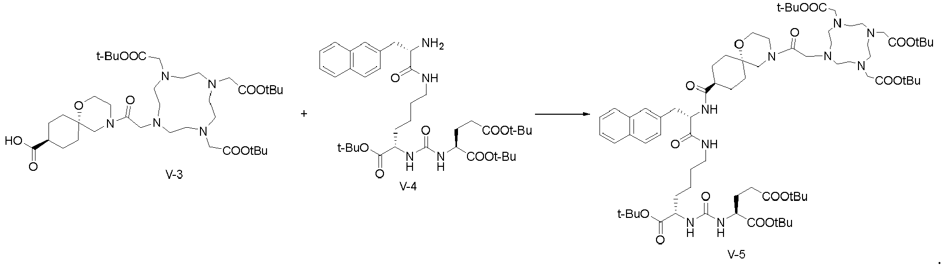

23. A preparation method for the compound according to any of claims 13 to 16, comprising the step of preparing the compound represented by formula (IV) or the pharmaceutically acceptable salt thereof according to any of claim 21 or 22, and further comprising the step of complexing the chelating agent in the compound represented by formula (IV) or the pharmaceutically acceptable salt thereof with the radionuclide.
